# EUROPEAN PATENT APPLICATION

(11) **EP 2 781 227 A1**
(43) Date of publication of application: **24.09.2014**
(21) Application number: 12849096.8
(22) Date of filing: 15.11.2012
(51) Int. Cl.: A61L 27/00

(54) **MEMBER FOR USE IN TISSUE REGENERATION, METHOD OF FORMING SAME, AND INK**

(30) Priority: 18.11.2011 JP 2011253214
(71) Applicant: Fujifilm Corporation, Minato-ku Tokyo 106-0031 (JP)
(72) Inventor: KASAI, Seishi, Ashigarakami-gun Kanagawa 258-8577 (JP)
(74) Representative: HOFFMANN EITLE
(86) International application number: PCT/JP2012/079715
(87) International publication number: WO 2013/073643

(57) **Abstract**

The present invention relates to a tissue regeneration member having a desired tissue regeneration capacity and a mechanical strength.

A tissue regeneration member of the present invention comprises a biodegradable resin film containing cytokine and calcium phosphate, and has a gradient structure in which a ratio between the calcium phosphate and the cytokine increases or decreases continuously in the thickness direction.

## Description

### Technical Field

The present invention relates to a tissue regeneration member used in the reproduction treatment, a method for forming the same, and an ink used in the method.

### Background Art

Conventionally, as a reproduction treatment of hard tissues (bone, teeth, etc.), the guided tissue regeneration method (GTR method) or the guided bone regeneration method (GBR method) has been known. In both methods, a blocking film which prevents invasion of tissues or cells that do not contribute to regenerating the corresponding tissues by securing spaces for tissue regeneration has been used. In the GTR method, its primary purpose is for spontaneous bone regeneration or regeneration of periodontal tissue as a whole, and the blocking function of the film is generally a period of 1 to 2 months. In the GBR method, the bone regeneration is guided actively by sealing an inducer such as bone graft, bone substitute materials or PRP in the space of bone regeneration and the blocking function of the film generally lasts for a period of about 6 months.

There are two kinds of blocking films, which are a non-absorbent blocking film and an absorbent blocking film, but there is an increasingly growing demand for the absorbent blocking film because its removal is unnecessary after tissue regeneration. As an absorbent blocking film, the biodegradable resins have generally been used.

For example, patent document 1 discloses a guided bone regeneration membrane having a two-layer structure of a nonwoven layer including a silicon-eluting type calcium carbonate and a biodegradable resin and on which an apatite is coated, and a nonwoven layer including a biodegradable resin. The former nonwoven layer has bone-forming ability, and the latter nonwoven layer has the function of blocking film which prevents the invasion of the soft tissues.

Further, Patent Document 2 discloses a dental treatment membrane with a two-layer structure of a gelatin hydrogel layer reinforced with a bioabsorbable material (blocking layer) and a gelatin hydrogel layer having a sustained-release drug function (tissue regeneration membrane).

### Prior Art Document

### Patent Document

[Patent Document 1] JP-A-2009-61109 (the term "JP-A" as used herein refers to an "unexamined published Japanese patent application")
[Patent Document 2] JP-A-2009-67732

### Summary of the Invention

### Problems that the Invention Is to Solve

While there is a convenience that the blocking film made of a biodegradable resin may be absorbed in vivo after the tissue regeneration and thus its removal work is not required, the mechanical strength of the film is weak, and the satisfactory therapeutic effect may not be obtained by the breakage of the membrane or the like.

In patent document 2, in order to solve this problem, the gelatin hydrogel layer of the blocking film is reinforced with a bioabsorbable material and again with a highly cross-linked gelatin hydrogel layer to maintain the mechanical strength, and as the inner layer, a low cross-linked gelatin hydrogel layer which has a drug release function is provided as a tissue regeneration membrane. In Patent Document 2, the rate of biodegradation of the blocking film is set to about six months, and the rate of biodegradation of the tissue regeneration membrane is set to about two months by the difference in degree of cross-linking of the gelatin hydrogel layer.

However, in the case of a two-layer structure of the Patent Document 2, the biodegradability becomes gradual, only the blocking film will be left two months later, and when the tissue regeneration is insufficient, the problem that the medical of interest may not be achieved occurs. In addition, in the highly cross-linked gelatin hydrogel film and the low cross-linked gelatin hydrogel film, the physical properties are very different. The distortion or the like is residual at the time of forming the film, and thus a strength problem still remains.

The present invention has been made in view of such circumstances, and an object thereof is to provide a tissue regeneration member having a desired tissue regeneration capacity and the mechanical strength, and a method for forming the same. Further, another object of the present invention is to provide an ink that can be used in the method of forming the tissue regeneration member.

### Means for Solving the Problems

Objects of the present invention have been achieved by the following means.
[1] A tissue regeneration member comprising a biodegradable resin film containing cytokine and calcium phosphate, and having a gradient structure in which a ratio between the calcium phosphate and the cytokine increases or decreases continuously in a thickness direction.
[2] The tissue regeneration member as described in the above [1],
   wherein, in the gradient structure, the degree of cross-linking of the biodegradable resin is changed continuously in the thickness direction, the degree of cross-linking of the biodegradable resin is high at a side where the ratio of the calcium phosphate to the cytokine is high, and the degree of cross-linking of the biodegradable resin is low at a side where the ratio of the calcium phosphate to the cytokine is low.
[3] The tissue regeneration member as described in the above [1] or [2],
   wherein the calcium phosphate is at least one kind of β-TCP, α-TCP, tetracalcium phosphate and hydroxyapatite.
[4] The tissue regeneration member as described in any one of the above [1] to [3],
   wherein the cytokine is at least one kind of bFGF, VEGF, TGF-β, G-CSF, EPO, BMP-2, TGF-β1, cell growth factor in platelet, and angiopoietin.
[5] The tissue regeneration member as described in any one of the above [1] to [4],
   wherein the calcium phosphate is a particle having an average particle size of 5 nm to 10 µm.
[6] A method of forming a tissue regeneration member as described in any one of the above [1] to [5], containing:
   discharging a first ink containing a biodegradable resin and cytokine and a second ink containing a biodegradable resin and calcium phosphate on a substrate by an inkjet method to form, on the substrate, a biodegradable resin film having a gradient structure in which a ratio between the calcium phosphate and the cytokine increases or decreases continuously in a thickness direction.
[7] The method of forming a tissue regeneration member as described in the above [6],
   wherein the ink-jet method uses at least a first inkjet head and a second inkjet head, the method containing:
   a step of supplying the first ink to the first inkjet head;
   a step of supplying the second ink to the second inkjet head;
   a controlling step of determining a ratio of an amount of the first ink discharged from the first inkjet head and an amount of the second ink discharged from the second inkjet head;
   a forming step of discharging the first ink or the second ink from at least one of the first inkjet head and the second inkjet head depending on the determined ratio to form one layer; and
   a laminating step of laminating a plurality of layers on the substrate through repeating the forming step to obtain a biodegradable resin film having the gradient structure,
   wherein, in the controlling step, the ratio is determined such that the ratio of the first ink becomes higher and the ratio of the second ink becomes lower from the closest layer towards the farthest layer from the substrate or from the farthest layer towards the closest layer to the substrate in the thickness direction of the plurality of layers.
[8] The method of forming a tissue regeneration member as described in the above [7],
   wherein an ink amount of droplets discharged from the first and second inkjet heads is from 0.3 pL to 100 pL.
[9] The method of forming a tissue regeneration member as described in the above [7] or [8],
   wherein a droplet diameter of droplets discharged from the first and second inkjet heads is from 1 µm to 300 µm.
[10] The method of forming a tissue regeneration member disclosed in the above [6], wherein the ink-jet method uses a plurality of inkjet heads, the method containing:
   a step of supplying a plurality of mixed inks including a first ink containing the first material and a second ink containing the second material which are mixed in different ratios, respectively, to each of the plurality of inkjet heads;
   a selecting step of selecting inkjet heads one by one sequentially from the plurality of inkjet heads, the selecting being made sequentially from an inkjet head to which a mixed ink having a high ratio of the first or second ink is supplied to an inkjet head to which a mixed ink having a low corresponding ratio is supplied;
   a forming step of discharging the mixed ink from the selected inkjet head to form a layer; and
   a laminating step of laminating a plurality of layers on the substrate through repeating the forming step to obtain a biodegradable resin film having the gradient structure.
[11] The method of forming the tissue regeneration member as described in the above [10],
   wherein an ink amount of droplets discharged from the first and second inkjet heads is from 0.5 pL to 150 pL.
[12] The method of forming the tissue regeneration member as described in the above [10] or [11],
   wherein a droplet diameter of droplets discharged from the first ink and the second inkjet head is from 2 µm to 450 µm.
[13] The method of forming the tissue regeneration member as described in any one of the above [6] to [12],
   wherein the first and second inks contain the biodegradable resin and the cytokine or the calcium phosphate, and the inks have a viscosity of 2 mPa·s to 50 mPa·s and a surface tension of 15 mN/m to 40 mN/m.
[14] An ink containing a biodegradable resin, and cytokine or calcium phosphate, wherein the ink has a viscosity of 2 mPa·s to 50 mPa·s and a surface tension of 15 mN/m to 40 mN/m.

### Advantage of the Invention

According to the present invention, a tissue regeneration member having a desired tissue regeneration capacity and mechanical strength can be provided. Further, a method of forming the tissue regeneration member and an ink used in the corresponding forming method can be provided.

### Brief Description of the Drawings

FIG. 1 is a schematic diagram of a tissue regeneration member made of a biodegradable resin film having a gradient structure.
FIG. 2 is a schematic diagram of a tissue regeneration member made of a biodegradable resin film having a gradient structure.
FIG. 3 is a diagram illustrating overall configuration of a manufacturing apparatus a biodegradable resin film having a gradient structure.
FIG. 4 is a schematic diagram of a manufacturing apparatus of a biodegradable resin film having a gradient structure.
FIG. 5 is a diagram for explaining the formation of a biodegradable resin film having a gradient structure by a drawing mixing method.
FIG. 6 is a diagram for explaining another embodiment of the drawing mixing method.
FIG. 7 is a diagram illustrating overall configuration of a manufacturing apparatus a biodegradable resin film having a gradient structure according to an embodiment of the ink mixing method.
FIG. 8 is a diagram for explaining the formation of a biodegradable resin film having a gradient structure by the ink mixing method.
FIG. 9 is a diagram for explaining the landing position of each ink in the drawing mixing method.

### Mode for Carrying Out the Invention

The tissue regeneration member of the present invention a tissue regeneration member which comprises a biodegradable resin film containing calcium phosphate and cytokine and has a gradient structure in which the ratio between the calcium phosphate and the cytokine increases or decreases continuously in the thickness direction

Since the ratio of a calcium phosphate to reinforce mechanically the biodegradable resin film and cytokine sustainedly released in accordance with the biodegradability of the biodegradable resin is changed so as to be inclined or increased continuously in the thickness direction, the membrane exhibits optimal tissue regeneration capacity, while maintaining the mechanical strength of the film. Thus, the reproduction treatment can be achieved in a high success rate.

The tissue regeneration member of the present invention can be suitably used as a member for hard tissue regeneration such as bones and teeth.

### <Biodegradable Resin Film>

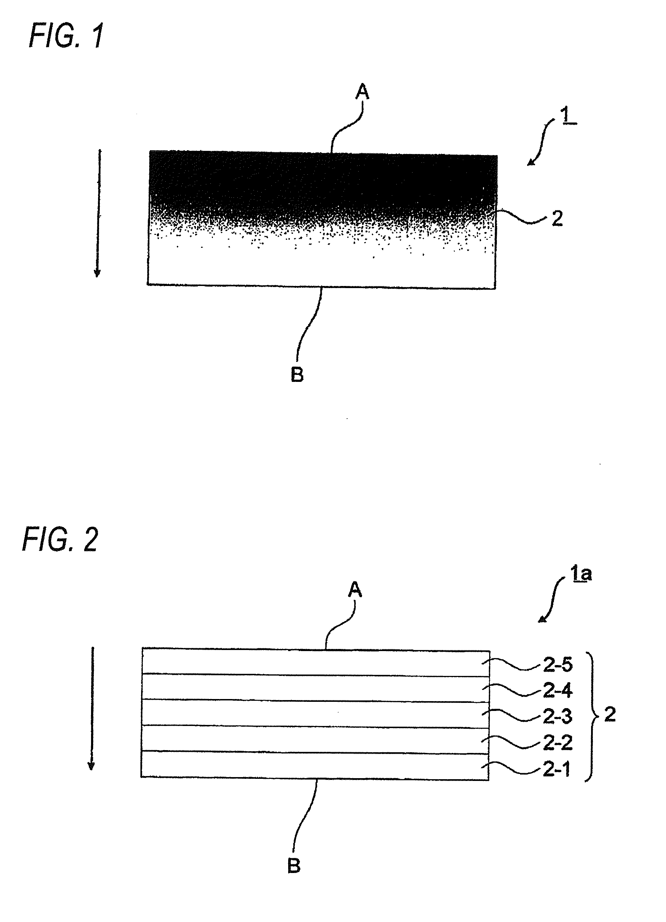

FIG. 1 shows schematically a cross section of a tissue regeneration member made of a biodegradable resin film having a gradient structure according to the present invention.

A tissue regeneration member 1 according to the present invention has a biodegradable resin film 2 in which a ratio between the calcium phosphate and the cytokine is changed continuously in a thickness direction (a direction of the arrow directed from the side A to the ide B in FIG. 1). Here, the term of "thickness direction" means the "film thickness direction" of the biodegradable resin film 2. Further, the expression of "the ratio between the calcium phosphate and the cytokine is changed continuously" means that when the thickness in the biodegradable resin film is divided into every regions having a certain thickness (for example, 1 µm to 5µm) in the thickness direction in consideration of the ratio of the calcium phosphate and cytokines in each region, the difference in the corresponding ratio between adjacent regions (when each ratio in the adjacent regions is set to R1 and R2, |R2-R1|) is 30% or less, preferably 10% or less, more preferably 7% or less, and even more preferably 5% or less. In addition, there may be regions in which the difference in ratio between the raw calcium phosphate and the cytokine between any two adjacent regions is zero.

In the biodegradable resin film 2, the ratio of the cytokine and the calcium phosphate in the vicinity of the surface on the one side of the thickness direction (for example, the ratio of the cytokine and the calcium phosphate in the region from A up to the thickness of 1 µm to 20 µm in FIG. 1) is that the ratio of the cytokine and the calcium phosphate (by mass ratio) is preferably from 1:500 to 1:10,000, more preferably from 1:750 to 1:7,500, and still more preferably from 1:1,000 to 1:5,000. Further, the ratio of the cytokine and the calcium phosphate in the vicinity of the surface on the opposite side of the thickness direction (for example, the ratio of the cytokine and the calcium phosphate in the region from B up to the thickness of 1 µm to 20 µm in FIG. 1) is that the ratio of the cytokine and the calcium phosphate (by mass ratio) is preferably from 1:25,000 to 1:250,000, more preferably from 1:50,000 to 1:200,000, and still more preferably from 1:100,000 to 1:150,000.

For example, the ratio of the cytokine and the calcium phosphate in each region can be determined by using high-sensitivity mass spectrometer and XPS in combination.

If there is a continuous change in the ratio of the cytokine and the calcium phosphate as described above, the configuration of the biodegradable resin film 2 is not particularly limited, but the configuration in which a plurality of layers having different ratios between the cytokine and the calcium phosphate calcium as illustrated in FIG. 2 are laminated may be mentioned as preferred examples.

A tissue regeneration member 1a illustrated in FIG. 2 has a biodegradable resin film 2, and the corresponding biodegradable resin film 2 has a plurality of layers 2-1, 2-2, 2-3, 2-4, 2-5 having a different ratio of the cytokine and the calcium phosphate calcium. In the layers 2-1, 2-2, 2-3, 2-4, 2-5, the ratio of the cytokine and the calcium phosphate is increased continuously from the layer 2-5 on a side A of the thickness direction toward the layer 2-1 of the opposite side B (i.e., in the direction of the arrow in FIG. 2).

In order to obtain a mechanical strength and a tissue regeneration capacity of a good film, in the layers 2-1, 2-2, 2-3, 2-4, 2-5, the difference in the ratio of the cytokine and the calcium phosphate in the two adjacent layers (when each ratio in the adjacent regions is set to R1 and R2, |R2-R1|) is 30% or less, preferably 10% or less, more preferably 7% or less, and even more preferably 5% or less. In addition, the ratio of the cytokine and the calcium phosphate in the layer 2-5 on the side A is preferably from 1:500 to 1:10,000, more preferably from 1:750 to 1:7,500, and still more preferably from 1:1,000 to 1:5,000. The ratio of the cytokine and the calcium phosphate in the layer (2-5) on the side B is preferably from 1:25,000 to 1:250,000, more preferably from 1:50,000 to 1:200,000, and still more preferably from 1:100,000 to 1:150,000.

In FIG. 2, the layers 2-1, 2-2, 2-3, 2-4, 2-5 are stacked so as to form the biodegradable resin film 2, but the number of layers to be stacked is not particularly limited. Preferably 3 to 10 layers and more preferably 3 to 7 layers are stacked. Further, the thickness of each layer is preferably 10 µm to 30µm, and more preferably 15 µm to 25µm. It is preferred that the thickness of each layer is substantially equal (errors of the thickness in the range of ± 5µm).

In the case the interface between the layers is not clear, the region which is separated by the thickness of 1 µm to 10µm in the thickness direction of the biodegradable resin film (2) may be considered as a "layer."

For example, the ratio of the cytokine and the calcium phosphate in each region can be determined by using high-sensitivity mass spectrometer and XPS in combination.

In the biodegradable resin film of the present invention as described above, it is possible to reinforce mechanically the biodegradable resin film by the calcium phosphate. Since the cytokine is sustained released according to the rate of biodegradation, the cytokine may promote tissue regeneration. The ratio of the calcium phosphate to reinforce mechanically the biodegradable resin film and the cytokine sustained released in accordance with the biodegradation of the biodegradable resin is changed so as to be gradient or increased continuously in the thickness direction, and thus, it is possible to exert the optimal tissue regeneration capacity while maintaining the mechanical strength of the membrane.

Here, the biodegradable resin having a low degree of cross-linking has in general a fast rate of biodegradation. Therefore, in order to control the rate of the sustained release of cytokines, it is preferred to vary the degree of cross-linking of the biodegradable resin in the thickness direction in the biodegradable resin film of the present invention.

That is, in the biodegradable resin film of the present invention, it is preferred that the degree of cross-linking of the biodegradable resin increases or decreases continuously in the thickness direction. Here, the expression of "the degree of cross-linking of the biodegradable resin increases or decreases continuously in the thickness direction" means that when the thickness in the biodegradable resin film is divided into every regions having a certain thickness (for example, 1 µm to 5 µm) in the thickness direction to look at the degree of crosslinking of the biodegradable resin in each region, the difference in the degree of cross-linking of the biodegradable resin between adjacent regions is 30% or less, preferably 10% or less, more preferably 7% or less, and even more preferably 5% or less, and the degree of cross-linking of the biodegradable resin between adjacent regions in the thickness direction is monotonically increasing or decreasing. If the difference between the degrees of cross-linking of the biodegradable resin between adjacent regions is larger than 10%, the change in the degree of cross-linking becomes gradual and the distortion between adjacent regions becomes large. Thus, there are cases where the high mechanical strength through the whole film cannot be obtained or a suitable biodegradation rate of the biodegradable resin cannot be obtained. Further, there may be regions in which the difference between the degrees of cross-linking of the biodegradable resin between any two adjacent regions is zero, but the difference it is preferably 1% or more.

The degree of cross-linking of the biodegradable resin in each region can be estimated in terms of the measurement of the swelling amount or water supply amount of the dry film.

In the biodegradable resin film of the present invention, it is preferred that the degree of cross-linking of the biodegradable resin is high at the side where the ratio of the calcium phosphate to the cytokine is high (side B in FIGS. 1 and 2), the degree of cross-linking of the biodegradable resin is low at the side where the ratio of the calcium phosphate to the cytokine is low (side A in FIGS. 1 and 2). In this case, the side where the degree of cross-linking of the biodegradable resin is low becomes a side facing the tissue to be reproduced so that the biodegradable resin is decomposed gradually according to changes in the degree of cross-linking, and thus the cytokine is slowly released and accordingly the tissue regeneration is promoted. The side where the degree of cross-linking of the biodegradable resin is high increases the mechanical strength of the film and also the biodegradation rate becomes slow so that a space for tissue regeneration is insured, and thus it can function as a blocking layer for preventing from invading of tissues and cells that do not contribute to the tissue regeneration.

In this case, in the biodegradable resin film 2, the degree of cross-linking of the biodegradable resin in the vicinity of the surface of one side of the thickness direction (for example, the degree of cross-linking of the biodegradable resin in the region up to 1 µm to 50 µm in thickness from A in FIG. 1) is preferably from 0% to 10%, more preferably 0% to 5%, and substantially more preferably 0% (0% to 1%). Further, the degree of cross-linking of the biodegradable resin in the vicinity of the surface on the opposite side (for example, the degree of cross-linking of the biodegradable resin in the region up to 1 µm to 50 µm in thickness from B in FIG. 1) is preferably 50% to 100%, more preferably 70% to 100%, and substantially more preferably 100% (99% to 100%).

Further, as for a configuration of the biodegradable resin film 2, in the configuration in which a plurality of layers are laminated as illustrated in FIG. 2, preferred examples include a configuration in which the degree of cross-linking of the biodegradable resin of each layer is different.

For example, in the tissue regeneration member 1a illustrated in FIG. 2, the layers 2-1, 2-2, 2-3, 2-4, 2-5 has a configuration in which the degree of cross-linking of the biodegradable resin is increased continuously from the layer 2-5 on the side A of the thickness direction toward the layer 3-1 of the opposite side B (i.e., in the direction of the arrow in FIG. 2) with in the range of 0% to 100%. In this case, the mechanical strength of a good film was maintained, and the suitable rate of biodegradation was obtained to give a desired tissue regenerative capacity, and in the layers 2-1, 2-2, 2-3, 2-4, 2-5, the difference of the degree of cross-linking of the biodegradable resin in two adjacent layers is 30% or less, preferably 10% or less, more preferably 7% or less, and still more preferably 5% or less. Further, the degree of cross-linking of the biodegradable resin in the layer (2-5) of the side A is preferably 0% to 10%, more preferably 0% to 5%, and far more preferably 0% to 1%. The degree of cross-linking of the biodegradable resin in the layer (2-1) of the side B is preferably 50% to 100%, more preferably 70% to 100%, and far more preferably 90% to 100%.

The degree of cross-linking of the biodegradable resin in each layer can be estimated in terms of the measurement of the swelling degree or water supply amount of the dry film.

### <Biodegradable Resin>

The biodegradable resin used in the present invention is not particularly limited, but examples thereof include homopolymers such as L-lactic acid, D-lactic acid, DL-lactic acid, glycolic acid, ε-caprolactone, N-methylpyrrolidone, trimethylene carbonate, paradioxanone, 1,5-dioxepane-2-one, hydroxy butiric acid, hydroxy valeric acid, acid anhydride (sebasic acid anhydride, maleic acid anhydride, oleic acid anhydride, etc.), glycine, alanine, phenylalanine, tyrosine, asparagine, glutamine, aspartic acid, glutamic acid, lysine, hydroxy lysine, arginine, valine, leucine, isoleucine, serine, threonine, cysteine, methionine, tryptophan, histidine, proline, hydroxyproline and other amino acids (L form, D form, a mixture of L form and D form), or the like, copolymers or mixtures of these polymers; polyacrylic acids such as polyesters, polycarbonates, poly(α-cyanoacrylate); polyphosphates, amino acid polymer compounds, polyacid anhydride, a protein (gelatin, etc.), polyglycosides (chitin, chitosan, starch , etc.), polysaccharides (alginate, carrageenan, etc.) and the like. The weight average molecular weight of the biodegradable resin is preferably 2,000 to 200,000, and more preferably from 20,000 to 500,000.

As examples of biodegradable resins, among others, from the viewpoints of versatility and safety, DL-lactic acid, glycolic acid, proteins, and polysaccharides (alginate, carrageenan) are preferred, polysaccharides and proteins are more preferred, and gelatin is far more preferred.

As the above mentioned gelatin, either those naturally obtained or those obtained by fermentation using microorganisms, chemical synthesis, or genetic recombination manipulation can be used. These materials can be used after suitably being mixed. Natural gelatin can be obtained from a variety of animal-derived collagen of pigs, cows, fish such as salmon, sea bream, shark, etc., including humans, by denaturing them through various processes of alkaline hydrolysis, acid hydrolysis, enzymatic degradation, etc.

In addition, gelatin derivatives which are modified gelatin may be used. Examples of gelatin derivatives include a chemical functional group such as a gelatin two azide group, a thiol group, an amino group, a carboxyl group, a sulfuric acid group and a phosphoric acid group, or hydrophobic residue such as alkyl group, an acyl group, a phenyl group, or a benzyl group, and groups in which compounds having these residues are introduced chemically, or a bioabsorbable oligomers, polymers, copolymers comprising lactic acid, glycolic acid, and the like, or water soluble oligomers such as polyethylene glycol, its copolymers with the propylene glycol, groups in which polymers are introduced chemically, and the like.

Preferred examples of the gelatin used in the present invention include acidic gelatin obtained by alkaline treatment of the collagen and its isoelectric point is in the acidic region or basic gelatin obtained by acid treatment of the collagen and its isoelectric point is in the alkaline range. Examples of the acidic gelatin and basic gelatin include those manufactured by Nitta Gelatin Co., Ltd., or Nippi Co., Ltd.

### < Calcium Phosphate >

The calcium phosphate used in the present invention is not particularly limited as long as it is possible to reinforce mechanically the biodegradable resin film and it is a bioabsorbable material, examples thereof include β-TCP, α-TCP, tetracalcium phosphate, hydroxyapatite and the like. Among these, for reasons of good regeneration-inducing suitability for bone and biocompatibility, β-TCP or hydroxyapatite is preferred, and β-TCP is more preferred.

The shape of calcium phosphate is not particularly limited, but from the viewpoint of ink suitability when forming the tissue regeneration member of the present invention by an inkjet method to be described later, suitability as a blocking layer and good tissue regeneration, a type of particles is also preferred. The average particle size preferably ranges from 5 nm to 10 µm, more preferably 10 nm to 7 µm, and still more preferably 10 nm to 5 µm.

The content of the calcium phosphate in the biodegradable resin film according to the present invention is preferably 5% by mass to 40% by mass, more preferably 10% by mass to 35% by mass, and still more preferably 15% by mass to 30% by mass.

### (Cytokine)

The cytokine used in the present invention includes preferably those that are sustained release due to the biodegradation of the biodegradable resin and have angiogenic potential. By angiogenesis, supply of nutrients and oxygen is carried out smoothly, and thus bone regeneration or the regeneration of periodontal tissue is possible in a short period of time. These may be obtained from natural materials or the materials produced synthetically. Examples thereof include bFGF, VEGF, TGF-β, G-CSF, EPO, BMP-2, TGF-β1, intra platelet cellular growth factor, angiopoietin, derivatives thereof, or mixtures thereof. Among these, because of the regeneration-inducing suitability of the hard tissue (bones, teeth, etc.), bFGF, VEGF, or BMP-2 is preferred, bFGF or VEGF is more preferred, and bFGF is far more preferred.

The content of cytokines in the biodegradable resin film according to the present invention is preferably 0.001% by mass to 0.1% by mass, more preferably 0.005% by mass to 0.05% by mass, and still more preferably is 0.005% by mass to 0.03% by mass.

### <Film Thickness>

The thickness of the biodegradable resin film according to the present invention is preferably 20 µm or more, more preferably 20 µm to 500 µm, and far more preferably 50 µm to 300 µm. Within this range, the space necessary for regeneration induction is ensured and the handling suitability is good.

### <Biodegradation Rate>

The biodegradation rate of the biodegradable resin film according to the present invention can be changed according to the changes of the degree of cross-linking in the thickness direction, and its speed can be adjusted according to the degree of cross-linking.

At the side where the degree of cross-linking is high (side B in FIGS. 1 and 2), the longer the rate of biodegradation is preferred in order to maintain the mechanical strength of the film, but it is more preferred that it is biodegraded rapidly after the tissue regeneration. The rate of biodegradation at the side where the degree of cross-linking is high (side B in FIGS. 1 and 2) is preferably 4 months to 8 months, and more preferably 5 months to 6 months.

On the other hand, at the side where the degree of cross-linking is low (side A in FIGS. 1 and 2), the faster the rate of biodegradation is preferred in order to slowly release the cytokine and to induce the tissue regeneration. The rate is preferably 0.5 month to 3 months, and more preferably 1 month to 2 months.

### <Method of Forming the Tissue Regeneration Member>

The method of forming the tissue regeneration member according to the present invention is not particularly limited as long as a film having a gradient structure in which the degree of cross-linking of the biodegradable resin increases or decreases continuously in the thickness direction may be formed, but an inkjet method is preferred from the viewpoint of easy fabrication of the gradient structure according to an affected part to which the tissue regeneration member must be applied and the shape and thickness of the tissue regeneration member can be arbitrarily controlled on demand.

A method for forming the tissue regeneration member using an ink-jet method will be explained below.

The method of forming the tissue regeneration member according to the present invention discharges a first ink containing a biodegradable resin and cytokine and a second ink containing a biodegradable resin and calcium phosphate on the substrate by an inkjet method to form a biodegradable resin film having a gradient structure in which a ratio between the calcium phosphate and the cytokine increases or decreases continuously in the thickness direction on the substrate. In this case, the above-mentioned gradient structure may be configured such that the degree of cross-linking of the biodegradable resin is increased or decreased continuously in the thickness direction at the same time.

### <Ink>

The ink according to the present invention (ink composition) contains a biodegradable resin, cytokine or calcium phosphate.

The method of forming the tissue regeneration member according to the present invention uses the first ink containing a biodegradable resin and a cytokine and the second ink containing a biodegradable resin and calcium phosphate. The first ink and the second ink may be used each independently, or both may be used as a mixed ink by mixing them.

Such an ink may contain solvent and other additives other than biodegradable resins, cytokine and calcium phosphate.

As a biodegradable resin, cytokine and calcium phosphate used in the first ink and the second ink, those described above can be used.

The content of the biodegradable resin in the first ink (the content based on the total amount of all the components except the solvent) is preferably 90% by mass to 99.9995% by mass, more preferably 95% by mass to 99.999% by mass, and still more preferably 98% by mass to 99.999% by mass.

The content of the cytokine in the first ink (the content based on the total amount of all the components except the solvent) is preferably 0.0005% by mass to 0.01% by mass, more preferably 0.001% to 0.007% by mass, and still more preferably 0.001% by mass to 0.005% by mass.

The content of the biodegradable resin in the second ink (the content based on the total amount of all the components except the solvent) is preferably 20% by mass to 80% by mass, more preferably 30% by mass to 70% by mass, and still more preferably 40% by mass to 60% by mass.

The content of the calcium phosphate in the second ink (the content based on the total amount of all the components except the solvent) is preferably 20% by mass to 80% by mass, more preferably 30% by mass to 70% by mass, and still more preferably 40% by mass to 60% by mass.

### <Cross-linking>

In the first ink or the second ink, a cross-linking agent may be used for the cross-linking of the biodegradable resin. In order to form the gradient structure in which the degree of cross-linking of the biodegradable resin increases or decreases continuously in the thickness direction by an inkjet method according to the present invention, it is preferred to use a cross-linking agent in the second ink.

Cross-linking agents include, for example, glutaraldehyde; calcium carbonate; water-soluble carbodiimide such as EDC; condensing agent to create a chemical bond between propylene oxide, diepoxy compound, a hydroxyl group, a carboxyl group, an amino group, a thiol group, an imidazole group, or the like. Glutaraldehyde can be preferably used.

The content of the cross-linking agent in the first ink is preferably 0% by mass to 10% by mass, more preferably 0% by mass to 5% by mass, and still more preferably 0% by mass to 2% by mass.

The content of the cross-linking agent in the second ink is preferably 3% by mass to 30% by mass, more preferably 5% by mass to 20% by mass, and still more preferably 7% by mass to 15% by mass.

In general, the degree of cross-linking of the biodegradable resin increases and the bioabsorbability is lowered as the concentration of the biodegradable resin and the cross-linking agent and cross-linking treatment time are increased. Therefore, it is possible to obtain the desired degree of cross-linking by adjusting these conditions.

Further, the biodegradable resin can also be crosslinked by the thermal dehydration treatment, or the irradiation of UV, gamma rays, electron beam, or the like, or by combining these cross-linking treatments.

### <Solvent>

In the present invention, the first ink and the second ink may be preferably prepared by mixing a biodegradable resin and cytokine or calcium phosphate with a solvent.

As a solvent, water or an organic solvent may be selected appropriately to be used. The liquid in which the boiling point is 50°C or more is preferred, and the organic solvent in which the boiling point is in the range of 60°C to 300°C is more preferred.

The solvent may be preferably used in a proportion of the solid content of the ink is 1% by mass to 50% by mass. Further, 5% by mass to 40% by mass is preferred. Within this range, the resulting ink is in the range of viscosity for good workability.

Examples of solvents include water, alcohols, ketones, esters, nitriles, amides, ethers, ether esters, hydrocarbons, halogenated hydrocarbons, and the like. Specifically, examples thereof include alcohols (e.g., methanol, ethanol, propanol, butanol, benzyl alcohol, ethylene glycol, propylene glycol, ethylene glycol monoacetate, cresol, etc.), ketones (e.g., methyl ethyl ketone, methyl isobutyl ketone, cyclohexanone, methyl cyclohexanone, etc.), esters (e.g., methyl acetate, ethyl acetate, propyl acetate, butyl acetate, ethyl formate, propyl formate, butyl formate, ethyl lactate, etc.), aliphatic hydrocarbons (e.g., hexane, cyclohexane), halogenated hydrocarbons (e.g., methylene chloride, methyl chloroform, etc.), aromatic hydrocarbons (e.g., toluene, xylene, etc.), amides (e.g., dimethylformamide, dimethylacetamide, n-methylpyrrolidone, etc.), ethers (e.g., dioxane, tetrahydrofuran, ethylene glycol dimethyl ether, propylene glycol dimethyl ether, etc.), ether alcohols (e.g., 1-methoxy-2-propanol, ethyl cellosolve, methyl carbinol, etc.), fluoro-alcohols (e.g., the compounds disclosed in paragraph [0020] of Japanese Patent Application Laid-Open No. H8-143709, paragraph [0037] of Japanese Patent Application Laid-Open No. H11-60807, etc.), or the like.

Such solvents may be used either alone or as a mixture of these two or more. Preferred examples of the solvent include water, toluene, xylene, methyl ethyl ketone, methyl isobutyl ketone, cyclohexanone, methanol, isopropanol, butanol, and the like.

### <Additives>

The first ink and the second ink according to the present invention may include other additives such as a surface tension adjusting agent, an antifouling agent, a water resistance imparting agent, a chemical resistance imparting agent, and the like. In particular, a surface tension adjusting agent may be preferably contained.

As a surface tension adjusting agent, acetylenic diol based surfactants may be preferably used, and specifically examples thereof include Surfynol 465, Surfynol 104 (or more, manufactured by Air Products and Chemicals, Inc., trade name), Olfine STG, Olfine E1010, Olfine PD002W (or more, manufactured by Nissin chemical industry Co., Ltd., trade name) or the like. The content of the surface tension adjusting agent in the first ink and the second ink according to the present invention is preferably 0.1% by mass to 3% by mass, more preferably 0.2% by mass to 2% by mass, and still more preferably 0.3% by mass to 1% by mass.

### <Physical Properties of Ink>

The viscosity of the first ink and the second ink according to the present invention is preferably 2 mPa·s to 50 mPa·s, more preferably 2 mPa·s to 40 mPa·s, and still more preferably 3 mPa·s to 30 mPa·s from the viewpoint of the uniformity of film formation, the stability of ink jet discharge, and the storage stability of the ink.

Further, the surface tension is preferably 15 mN/m to 40 mN/m, more preferably 20 mN/m to 40 mN/m, and still more preferably 25 mN/m to 35 mN/m, from the viewpoint of the uniformity of film formation, the stability of ink jet discharge, and the storage stability of the ink.

### <Substrate>

In the method of forming the tissue regeneration member of the present invention, a biodegradable resin film having the gradient structure is formed on a substrate by an inkjet method. After forming the film, the biodegradable resin film is generally peeled off from the substrate and then used.

Therefore, as the base material, an organic based, an inorganic based, or a metallic based material is all available, but it is preferred to peel off easily the biodegradable resin film after film formation.

In particular, examples thereof include quartz glass, alkali-free plate glass, and the like, or synthetic resins manufactured such as polyethylene, polypropylene, polyvinyl chloride, polystyrene, polyvinyl acetate, acrylic resins, polyamide, polyacetal, polycarbonate, modified polyphenylene ether, polybutylene terephthalate, polyethylene terephthalate, and the like.

The surface of the substrate is preferably smooth, and the value of the average roughness Ra is preferably less than 1µm, and more preferably less than 0.8 µm, and still more preferably less than 0.7 µm.

### <Formation of Biodegradable Resin Film by the Inkjet Method>

Hereinafter, the formation of the biodegradable resin film by the ink-jet method of the present invention will be explained.

In the present invention, the present invention discharges the first ink containing a biodegradable resin and cytokine and the second ink containing a biodegradable resin and calcium phosphate on the substrate by an inkjet method as an ink of two or more separated respectively, or discharges a mixed ink made by mixing the first ink and the second ink on the substrate by an ink-jet method.

As an inkjet method, if the method is to perform an image recording by an inkjet printer, there is no limitation on the recording way of ink-jet. Any known manner, for example, a charge control method to discharge the ink composition by using an electrostatic attraction force, a drop on demand method using vibration pressure of the piezoelectric element (pressure pulse method), an acoustic inkjet method of discharging the ink composition by converting the electrical signal to acoustic beam to irradiate it on the substrate and discharging the ink composition using the radiation pressure, and a thermal inkjet (bubble jet (registered trademark)) method by heating the ink composition to form bubbles and by utilizing the pressure caused, and the like.

The control of the ink droplet is mainly carried out by a print head. For example, in the case of a thermal inkjet method, controlling the droplet discharge volume by the structure of the print head is possible. That is, by changing the size of the ink chamber, heating section and the nozzle, it is possible to discharge droplets in the desired size. In addition, even in a thermal inkjet method, by having a plurality of print heads in which the sizes of the heating unit or the nozzle are different, it is possible to realize the droplet discharge of a plurality of sizes. In the case of a drop-on-demand method using a piezoelectric element, it is possible to change the droplet discharge volume of the structure of the print head in the same way to the thermal ink jet system, but also by controlling the waveform of the drive signal for driving the piezoelectric element, it is possible to perform the droplet discharge of a plurality of sizes by the print head of the same structure.

An example of the method of discharging an ink onto a substrate (drawing method) include a drawing mixing method of supplying a first ink containing a biodegradable resin and cytokine and a second ink containing a biodegradable resin and calcium phosphate to a separate ink jet head and discharging simultaneously both while adjusting the ratio of the discharge amount of both to mix them on the substrate. Further, another separate method includes a mixed ink method of supplying a plurality of kinds of ink prepared in that the ratio of both are different in a mixed ink of mixing a first ink containing a biodegradable resin and cytokine and a second ink containing a biodegradable resin and calcium phosphate in advance to an ink jet head, selecting the head in order, and discharging successively a mixed ink in which the ratio of the first ink containing a biodegradable resin and cytokine and the second ink containing a biodegradable resin and calcium phosphate is different to be drawn.

### <Preparation of Ink>

The preparation of a first ink containing a biodegradable resin and cytokine and a second ink containing a biodegradable resin and calcium phosphate used in the drawing mixing method to be described later will be explained.

Each of the above-mentioned ink may be prepared by mixing the materials of each ink. When mixing the respective materials, it may be stirred by a stirrer. The stirring time is not particularly limited, but usually from 30 minutes to 60 minutes, and preferably 30 minutes to 40 minutes. Also, the temperature at the time of mixing is usually 10°C to 40°C, and preferably 20°C to 35°C.

In the ink mixing method to be described later, it is possible to use the prepared ink after mixing as described above.

### <Drawing Mixing Method>

The above-mentioned drawing mixing method is preferably a method of forming a biodegradable resin film having a gradient structure in which a ratio between the calcium phosphate and the cytokine increases or decreases continuously in a thickness direction on the substrate by discharging the first ink containing a biodegradable resin and cytokine and the second ink containing a biodegradable resin and calcium phosphate on the substrate by an inkjet method, the method including:
a step of supplying the first ink to the first inkjet head;
a step of supplying the second ink to the second inkjet head;
a controlling step of determining a ratio of an amount of the first ink discharged from the first inkjet head and an amount of the second ink discharged from the second inkjet head;
a forming step of discharging the first ink or the second ink from at least one of the first inkjet head and the second inkjet head depending on the determined ratio to form one layer; and
a laminating step of laminating a plurality of layers on the substrate through repeating the forming step to obtain a biodegradable resin film having the gradient structure,
in which, in the controlling step, the ratio is determined such that the ratio of the first ink becomes higher and the ratio of the second ink becomes lower from the closest layer towards the farthest layer from the substrate or from the farthest layer towards the closest layer to the substrate in the thickness direction of the plurality of layers.

According to the drawing method, it is possible to form a biodegradable resin film having a gradient structure in which the ratio of cytokine and calcium phosphate increases or decreases continuously in the thickness direction by using the techniques of inkjet system, comprising a step of determining the ratio of the discharge amount of the first ink discharged from the first inkjet head and the discharge amount of the second ink discharged from the second inkjet head, and a step of laminating a plurality of layers on the substrate by repeating the step of forming one layer by discharging ink according to the determined ratio, wherein, if one of the plurality layers goes to the upper layer, the ratio of the discharge amount of the first ink is larger and the discharge amount of the second ink is smaller. In addition, at the same time, the degree of cross-linking of the biodegradable resin may be increased or decreased continuously in the thickness direction in the gradient structure.

### <An embodiment of the drawing mixing method>

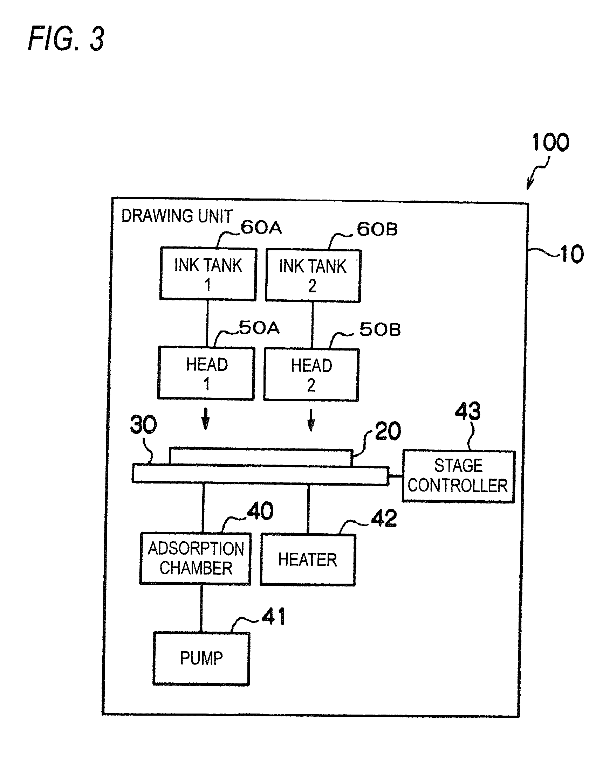

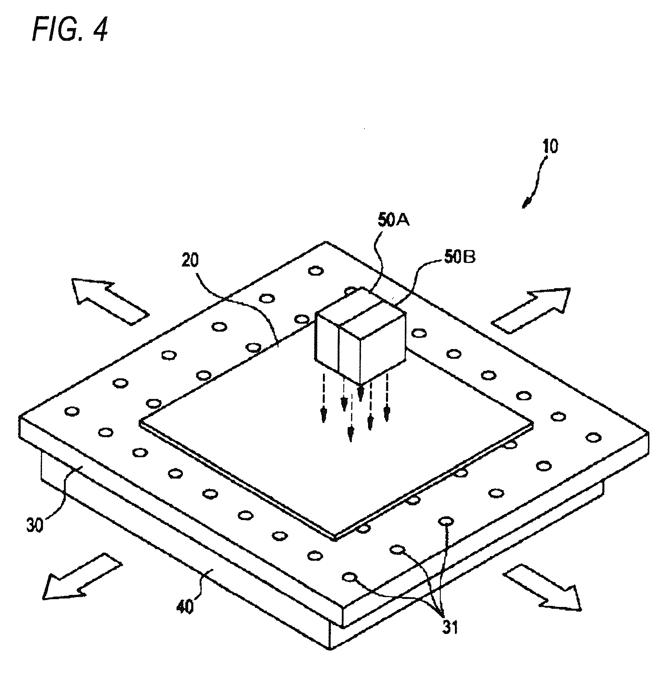

FIG. 3 is a diagram illustrating overall configuration of a manufacturing apparatus 100 of a biodegradable resin film having a gradient structure by the drawing mixing method, and FIG. 4 is a schematic diagram of a drawing unit 10 of the manufacturing apparatus 100. As illustrated in these figures, the manufacturing apparatus 100 is configured to include a drawing unit 10, and the drawing unit 10 employs a flat-bed type inkjet drawing device. In particular, the drawing unit 10 is configured to include a stage 30 configured to place a substrate, an adsorption chamber 40 configured to adsorb and hold the substrate placed on the stage 30, and an inkjet head 50A (hereinafter, referred to as an inkjet head 1) and an inkjet head 50B (hereinafter, referred to as an inkjet head 2) configured to discharge an ink toward the substrate 20.

The stage 30 has a width greater than the diameter of the substrate 20, and is configured to be freely movable in the horizontal direction by a moving mechanism (not illustrated). As a moving mechanism, for example, it is possible to use rack-and-pinion mechanism, a ball screw mechanism, or the like. The stage controller 43 (not illustrated in FIG. 4) can move the stage 30 to a desired position by controlling the moving mechanism.

Further, a number of the suction holes 31 are formed on the substrate holding surface of the stage 30. The adsorption chamber 40 is provided on the lower surface of the stage 30, and the substrate 20 is adsorbed and held on the stage 30, as the adsorption chamber 40 is evacuated by a pump 41 (not illustrated in FIG. 4). In addition, the stage is provided with a heater 42 (not illustrated in FIG. 4), and is able to heat the substrate 20 adsorbed and held on the stage 30 by the heater 42.

The inkjet heads 1 and 2 discharge an ink supplied from an ink tank 60A (hereinafter, referred to as an ink tank 1) and an ink tank 60B (hereinafter, referred to as an ink tank 2) to a desired position of a transparent support 20, where a head with a piezoelectric type actuator is used in this case. The inkjet heads 1 and 2 are disposed respectively as close as possible and fixed by a fixing means (not illustrated).

The inks supplied from the ink tanks 1 and 2 to the inkjet heads 1 and 2 are set to be the ink 1, and the ink 2, respectively. In the present invention, a first ink (hereinafter, referred to as a "guided tissue regeneration layer forming ink") containing a biodegradable resin and cytokine is set to be an ink 1, and a second ink (hereinafter, referred also to as a "tissue blocking layer forming ink") containing a biodegradable resin and calcium phosphate is set to be an ink 2.

### <Preparation of the biodegradable resin film having a gradient structure by drawing mixing method>

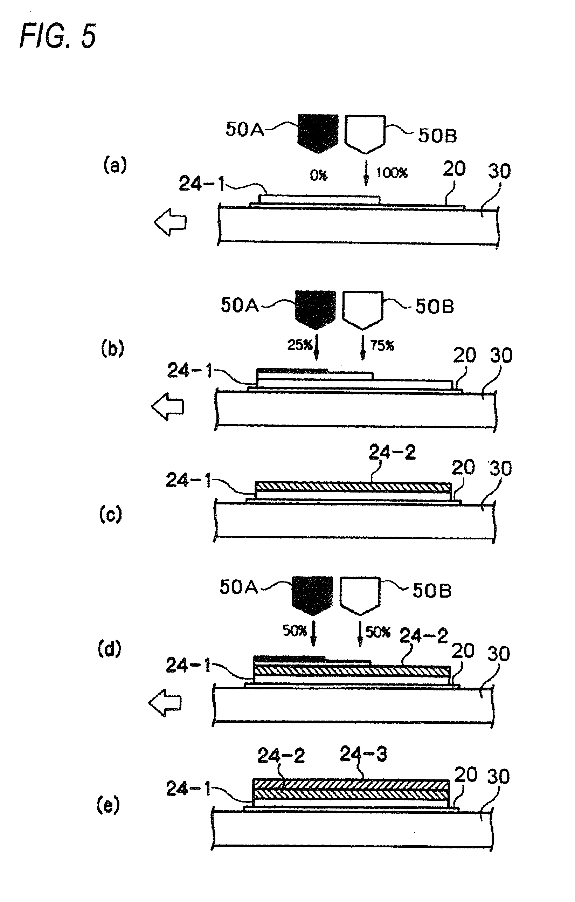

Referring to FIG. 5, description will be made on formation of the biodegradable resin film having a gradient structure in which the ratio between the cytokine and the calcium phosphate and the degree of cross-linking of the biodegradable resin increases or decreases continuously in the thickness direction by using the manufacturing apparatus 100 prepared as described above.

First, the substrate 20 is disposed on the stage 30 of the drawing unit 10 placed under a nitrogen atmosphere. The substrate 20 is placed such that the rear surface thereof is in contact with the stage 30. The adsorption of the substrate 20 to the stage 30 and heating are carried out by the adsorption chamber 40. In this case, substrate 20 is preferably heated to 70°C.

Next, one or several layers of the ink (ink 2) supplied from the inkjet head 2 are laminated on the adsorbed and heated substrate 20 to form a layer 24-1. This lamination of the ink 2 is carried out by discharging the ink 2 by the inkjet head 2 while moving the stage 30 by a moving mechanism (moving toward the left in the figure), as illustrated in FIG. 5(a). In this case, the discharge of the ink from the inkjet head 1 is not carried out.

It is preferred that the layer 24-1 of the ink 2 thus formed is semi-dried (semi-cured) to the extent that the biodegradable resin in the ink 2 is not completely cross-linked. Specifically, drying is carried out with less energy than the energy applied when dried normally (total dry (completely cured)).

In the present invention, it is preferred that a process of semi-drying the layer discharged in the forming step is carried out as described above. For example, for a semi-dry process, it is preferred to maintain at an environmental temperature of 40°C to 120°C for a predetermined period of time after the discharge of ink, and it is more preferred to maintain at an environmental temperature of 50°C to 100°C for a predetermined period of time. The corresponding maintenance time is preferably is 10 seconds to 120 seconds, and more preferably 20 seconds to 90 seconds.

Then, a mixed layer 24-2 of the ink 1 and the ink 2 is formed on the layer 24-1 of ink 2 which became the semi-dried state. The formation of the mixed layer 24-2 is performed by discharging the ink 1 by the inkjet head 1 while moving the stage 30, and at the same time by discharging the ink 2 by the inkjet head 2 as illustrated in FIG. 5(b). At this time, a discharge amount of the ink 1 and a discharge amount of the ink 2 are adjusted to the desired ratio. Here, the discharge amount of the ink 2 is set to be 75% and the discharge amount of the ink 1 is set to be 25% by adjusting the discharge amount of each nozzle of the inkjet heads 1 and 2. Further, the term, "the discharge amount" of ink in the present specification means the total amount of ink discharged to form each layer. Meanwhile, a "droplet amount" of the ink droplet discharged from the inkjet head to be described later means an amount of one ink droplet.

In addition, the adjustment of the ratio of a discharge amount of ink from the inkjet heads 1 and 2 may be adjusted by a dot pitch density of the drawing. For example, it is also possible to perform the adjustment of the ratio of the discharge amount by controlling the number of nozzles of the inkjet heads 1 and 2 for discharging inks to be 75:25 while maintaining the discharge amount of individual nozzles of the inkjet heads 1 and 2 constant.

After ink discharge, as illustrated in FIG 5(c), a mixed layer 24-2 is laminated by diffusing and mixing the ink 1 and the ink 2 discharged in each discharge amount. Since the layer 24-1 of the ink 1 became a semi-dried state, the solvent of the ink of the mixed layer 24-2 formed thereon is received in the layer 24-1 of the ink, and thus there is no extreme leakage and spreading. That is, the heating temperature by the heater 42 needs to be adjusted with the ease of evaporation of the ink. Depending on the type of solvent, the drawing may be performed at a temperature lower than 70°C as described above, for example, by setting the temperature of the substrate to about 50°C.

That is, in the above-mentioned forming process, it is preferred to have a step of diffusing and mixing the first ink and the second ink discharged. An example of the diffusing and mixing method includes a method of utilizing convection by heating or a method of using ultrasonic waves.

Further, two inkjet heads are arranged to be as close as possible, only one ink is dried and it is prevented that the mixing within the layers of both the ink is insufficient. Moreover, when discharging the two inks at the same time, and the droplets of the ink 1 discharged from the inkjet head 1 and the droplets of the ink 2 discharged from the inkjet head 2 may be collided in the air, mixed and landed.

Further, as described in more detail later, two inkjet heads were configured to have a width larger than the width (shorter side) of a target substrate respectively and to form one layer in a single scanning. Thus, the ink 1 and the ink 2 are easily being mixed.

Further, in order to facilitate mixing of the ink, the substrate 20 may be ultrasonic treated by controlling the stage 30. At this time, in order not to generate nodes by ultrasonic wave, it is preferred that the frequency of the ultrasonic waves is swept or the position of the substrate 20 is changed.

When the formed mixed layer 24-2 is in a semi-dried state in the same manner as in the layer 24-1 of the ink 2, the ratio of the amount of the mixed layer 24-2 is 25:75, and is in a state that the biodegradable resin and the calcium phosphate contained in the ink 2 and the biodegradable resin and the cytokine contained in the ink 1 are mixed and stacked.

Then, a mixed layer 24-3 is formed on the mixed layer 24-2. The formation of the mixed layer 24-3, as illustrated in FIG. 5(d), ink is discharged at the same time while moving the stage 30 by the inkjet head 2 and the inkjet head 1. The formation of the mixed layer 24-3 is performed by discharging simultaneously the ink by inkjet head 1 and the inkjet head 2 while moving the stage (30), as illustrated in FIG. 5(d). Here, the ink 1 and the ink 2 are both discharged at a ratio of discharge amount of 50%.

Since the mixed layer 24-2 is also in a semi-dried state, the solvent of the ink in the mixed layer 24-3 formed thereon is received in the mixed layer 24-2. After ink discharge, the mixing layer 24-3 is stacked by diffusing and mixing the two inks as illustrated in FIG. 5(e).

In addition, the mixed layer 24-3 is also semi-dried in the same manner as the layer 24-1 of ink 2. The ratio of the amount of the mixed layer 24-3 is 50:50, and it is in a state that the biodegradable resin and the cytokine contained in the ink 1 and the biodegradable resin and the calcium phosphate contained in the ink 2 are mixed and stacked.

Thus, the ratio of the discharge amount of the ink 1 and the ink 2 may be changed stepwise (as gradient) to form each of the mixed layers, and finally to form a layer in which the discharge amount of the ink 1 is 100%.

After the completion of the formation of all the layers, the diffusion of each layer proceeds, and the layer formed stepwise becomes a continuous layer. After the completion of the formation of all the layers, for a full-dry process, for example, it is preferred to maintain at an environmental temperature of 40°C to 120°C for a predetermined period of time after the discharge of ink, and it is more preferred to maintain at an environmental temperature of 50°C to 100°C for a predetermined period of time. The corresponding maintenance time is preferably 10 seconds to 120 seconds, and more preferably 20 seconds to 90 seconds. Here, the ratio of the cytokine and the calcium phosphate in each layer is different. As a result, as illustrated in FIG. 1, a biodegradable resin film 2 having a composition ratio in which 100% of the ink 2 is changed to 100% of the ink 1 through the side B to the side A in the film thickness direction is formed. Accordingly, a biodegradable resin film 2 having a gradient structure in which the ratio of the calcium phosphate to the cytokine is changed continuously from 100% to 0% from the side B to the side A. Further, since the concentration of the cross-linking agent in each layer is different, the degree of cross-linking of the biodegradable resin is different in each layer after a full-cure. As a result, in the biodegradable resin film 2, the gradient structure in which the degree of cross-linking of the biodegradable resin is changed continuously from 100% to 0% from the side B to the side A is obtained.

Thus, by setting the lower layer in a semi-dried state and forming the upper layer thereon, the diffusion may proceed to some extent between the upper layer and the lower layer. At this time, it is preferred not to be in a state where the interface between the upper and lower layers is lost, and the upper and lower layers are completely mixed and are not distinguished from each other.

In addition, after the completion of the formation of each layer, it is also possible to laminate dummy pattern in a region where the biodegradable resin film does not work and to measure the height of the dummy pattern by the optical displacement sensor or the like using a laser. In the state where the drying does not proceed and the solvent remains, since the film thickness is increased, it is possible to detect the dry state by the height of the dummy pattern.

As described above, it is possible to form a biodegradable resin film having the gradient structure by using an inkjet head. According to the drawing mixing method of the present embodiment, regardless of the number of layers to be formed, there is an advantage that the type of ink and the number of the inkjet heads can be reduced. The mixed layer of the ink 1 and the ink 2 may be stacked in multiple layers so that the mixing ratio of each ink is inclined stepwise.

Further, in the step of forming each layer, from the viewpoint of film thickness control and the fine wire formability, the amount of ink droplets discharged from the first inkjet head and the second inkjet head is preferably from 0.3 pL to 100 pL, more preferably from 0.5 pL to 80 pL, and still more preferably from 0.7 pL to 70 pL.

In the step of forming each layer, from the viewpoint of film thickness control and the fine wire formability, the diameter of ink droplets discharged from the first inkjet head and the second inkjet head is preferably from 1 µm to 300 µm, more preferably from 5 µm to 250 µm, and still more preferably from 10 µm to 200 µm.

Further, in the step of forming each layer, about the ink in which the ratio of the discharge amount is smaller among the first ink and the second ink, at least one of the droplet amount of the ink droplets discharged from the inkjet head and the droplet diameter is preferably set to be smaller than the ink in which the ratio is larger. For example, the ink droplets in which the ratio is small is preferably 0.3 pL to 60 pL, and the ink droplets in which the ratio is large is preferably 1 pL to 100 pL ink. Thus, diffusing and mixing time can be shorten, or the uniformity of the mixing can be improved.

In addition, the term of "droplet diameter" of the ink droplets means the length of the droplet diameter, and it is measured from the photo of a flight state when discharged from inkjet.

In the present embodiment, a biodegradable resin film 2 in which 100% of the ink 2 is changed to 100% of the ink 1 through the side B to the side A is formed, but there is no need to form a film so that the ink 2 or the ink 1 in the side B or side A is 100%. As long as the range is to obtain a biodegradable resin film 2, it is possible to change the ratio of the ink 2 or the ink 1 in the side B or the side A arbitrarily.

The ratio of the ink 2 or the ink 1 in the side A or the side B may be appropriately adjusted by the characteristics of the biodegradable resin film 2 such as adhesion or the biodegradation rate or the like to be obtained.

Further, in the present embodiment, the inkjet head 1 and the inkjet head 2 discharge inks simultaneously to form each layer, but may discharge inks sequentially.

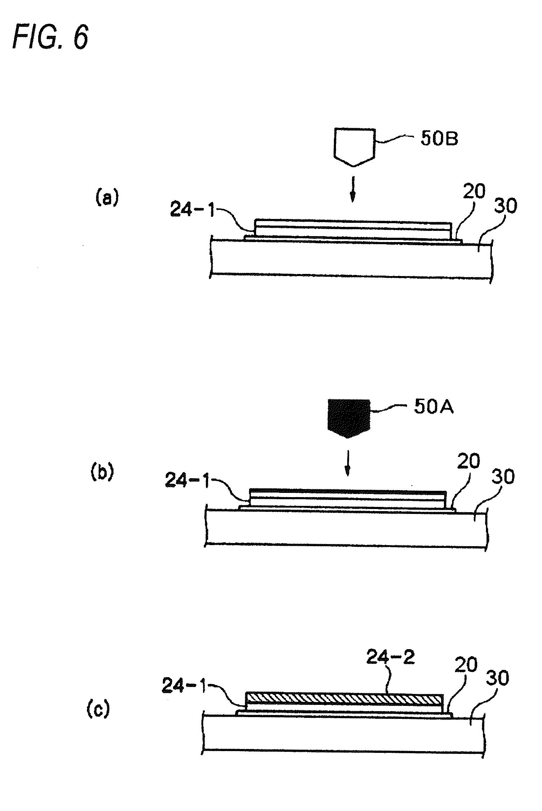

For example, in the case of forming the mixed layer 24-2, as illustrated in FIG. 6(a), first, the ink 2 is discharged onto the whole surface above the layer 24-1 of the ink 2 by the inkjet head 2. Next, as illustrated in FIG. 6(b), the ink 1 is discharged onto the whole surface by the inkjet head 1. After that, as illustrated in FIG. 6(c), it is possible to form a mixed layer 24-2 by diffusing and mixing each ink in the same manner.

Thus, in a case of forming one layer by discharging in turn each ink, when there is a difference between the discharge amounts of the two inks, that is, the ratio of the discharge amounts of the two inks is not 50% each, it may be configured to first discharge an ink with the larger discharge amount. In particular, when the ink that is discharged first is too dried, the less the amount is, the faster the drying is. Thus, it is preferred that the ink with the larger discharge amount may be discharged first. Accordingly, the mixing of two kinds of inks may be proceeded smoothly.

Further, in this case, for the ink having the smaller discharge amount that would otherwise be discharged at a later time, the small droplets (having a small droplet amount or a small droplet diameter) may be discharged by increasing the dot pitch density. Thus, it is possible to shorten the time for diffusing and mixing.

Further, it is preferred to land the ink to be discharged later overlappingly on the position where the ink discharged earlier is landed. In particular, in the case where the dots are separated from each other by performing intermittent striking, if landing on the same position prior to drying, the mixing of each ink is facilitated.

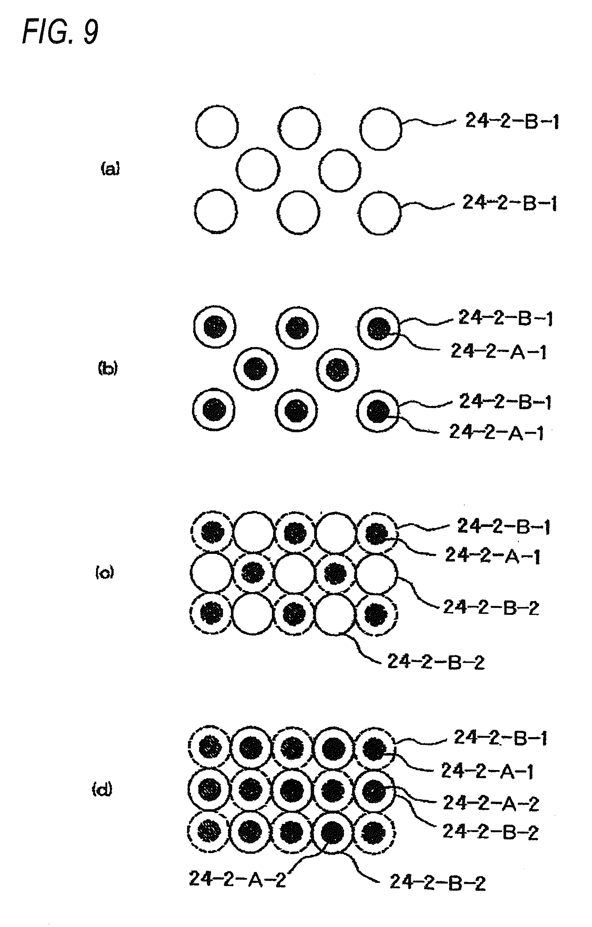

For example, when forming a mixed layer 24-2, it is said that the ink 2 is discharged by intermittent striking by the inkjet head 2 in the first scanning. FIG. 9(a) illustrates the ink 2 (24-2-B-1) landed on the layer 24-1 of the ink 1.

Then, the ink 1 is discharged by intermittent striking by inkjet head 1 in the second scanning. At this time, as illustrated in FIG. 9(b), the inkjet head 1 discharges the ink 1 (24-2-A-1) to land it overlappingly on the same position as the ink 2 (24-2-B-1) landed in the first scanning.

Further, the ink 2 is intermittently struck by the second inkjet head in the third scanning. FIG. 9(c) illustrates the ink 2 (24-2-B-2) landed between the inks 2 (24-2-B-1).

Then, in the fourth scanning, the ink 1 is discharged by the inkjet head 1 to be landed overlappingly on the same landing position as the ink 2 (24-2-B-2). As illustrated in FIG. 9(d), the ink 1 (24-2-A-2) is discharged to be landed overlappingly on the same position as the ink 2 (24-2-B-2) landed in the second scanning.

After that, equally, the ink is discharged onto the entire surface of the layer 24-1 of the ink 1, and then diffused and mixed.

By discharging in this manner, it is possible to shorten the time for diffusing and mixing when forming the mixed layer 24-2.

Further, if the drying of one ink is fast, the ink may be discharged later.

Further, in the present embodiment, the respective mixed layers were formed by using two pure inks of the ink 1 and the ink 2, but an ink mixture thereof may be used in combination. For example, it is contemplated that a mixed layer may be formed by simultaneously using three kinds of inks, that is, two pure inks and the mixed ink in which the mixing ratio of the ink 1 and the ink 2 is 50:50. The number of the inkjet head is increased by the amount of the mixed ink, but since the mixed ink has two pure inks mixed sufficiently in advance, it is possible to shorten the time required for diffusing and mixing of the ink after its discharge.

### <Ink mixing method>

The above-mentioned ink mixing method is preferably a method of forming a biodegradable resin film having a gradient structure in which a ratio between the calcium phosphate and the cytokine increases or decreases continuously in a thickness direction on the substrate by discharging a first ink containing a biodegradable resin and cytokine and a second ink containing a biodegradable resin and calcium phosphate on the substrate by an inkjet method, the method including:
a step of supplying a plurality of mixed inks including the first ink containing the first material and the second ink containing the second material which are mixed in different ratios, respectively, to each of the plurality of inkjet heads;
a selecting step of selecting inkjet heads one by one sequentially from the plurality of inkjet heads, the selecting being made sequentially from an inkjet head to which a mixed ink having a high ratio of the first or second ink is supplied to an inkjet head to which a mixed ink having a low corresponding ratio is supplied;
a forming step of discharging the mixed ink from the selected inkjet head to form one layer, and
a laminating step of laminating a plurality of layers on the substrate through repeating the forming step to obtain a biodegradable resin film having the gradient structure.

According to the above-mentioned method, since a plurality of inks in which the first ink and the second ink are mixed in different ratios are supplied to each of the inkjet head, the mixed inks are discharged sequentially from an inkjet head to which a mixed ink having a low ratio of the first ink is supplied so as to form each layer and stack a plurality of layers on the substrate, it is possible to form a biodegradable resin film having a gradient structure in which the ratio of the cytokine and the calcium phosphate increases or decreases continuously in the thickness direction, by using an inkjet type technique. In addition, simultaneously, the degree of cross-linking of the biodegradable resin increases or decreases continuously in the thickness direction in the corresponding gradient structure.

<An embodiment by ink mixing method>

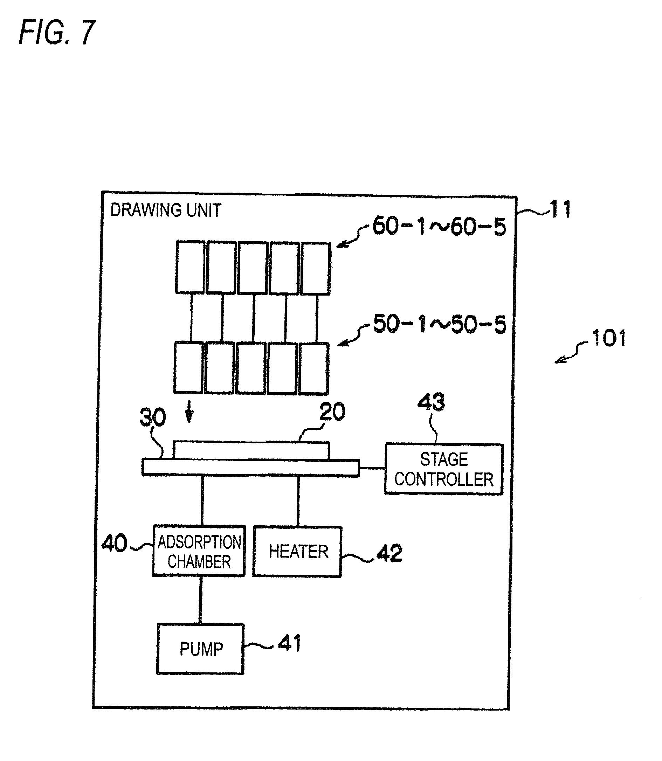

FIG. 7 is a diagram illustrating the overall configuration of a manufacturing apparatus 101 of the biodegradable resin film according to the second embodiment. As illustrated in the same figure, the manufacturing apparatus 101 of the biodegradable resin film according to the present embodiment is provided with a drawing unit 11, the drawing unit 11 is provided with ink tanks 60-1 to 60-5 for storing 5 kinds of inks and inkjet heads 50-1 to 50-5 to which the inks are supplied from each ink tank. Each of the inkjet heads 50-1 to 50-5 discharges an ink supplied from each of the ink tanks 60-1 to 60-5 to the substrate 20.

The inks supplied from each of the ink tanks 60-1 to 60-5 to each inkjet head 50-1 to 50-5 are set such that the mixing ratio of the ink 1 and the ink 2 is 0:100, 25:75, 50:50, 75:25, 100:0, respectively. That is, the pure ink of the ink 2 is supplied from the ink tank 60-1, the pure ink of the ink 1 is supplied from the ink tank 60-5, and the mixed inks in which the ink 1 and the ink 2 are mixed in a predetermined ratio are supplied from the ink tanks 60-2 to 60-4.

### <Preparation of biodegradable resin film having a gradient structure by the ink mixing method>

In the same manner as in the embodiment by the drawing mixing method, and placing the substrate 20 was placed on the stage 30, adsorbed and heated.

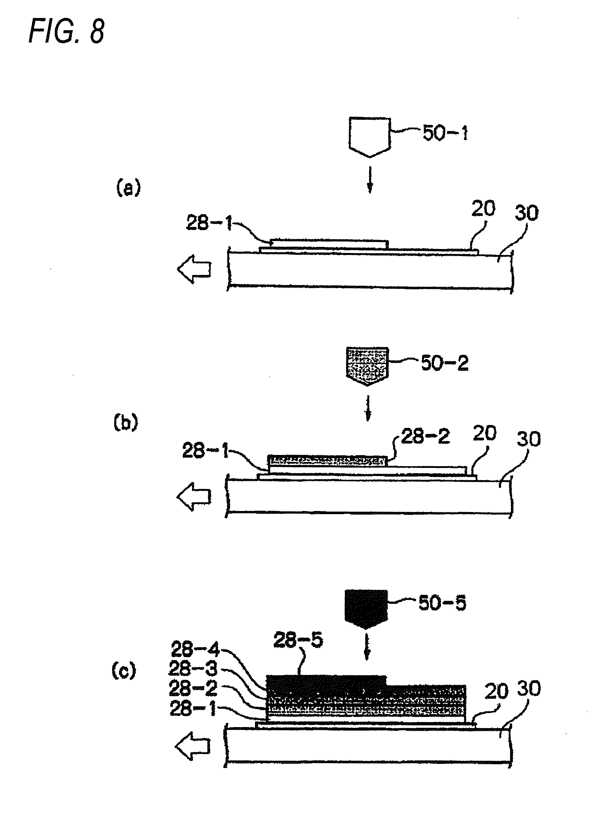

Next, one or several layers of the ink 2 are laminated on the adsorbed and overheated substrate 20 to form a layer 28-1 of the ink 2. This lamination of ink 2 is carried out by discharging an ink supplied from the ink tank 60-1 (an ink in which the mixing ratio of the ink 1 and the ink 2 is 0:100) on the substrate by the inkjet head 50-1 while moving the stage 30 by a moving mechanism (moving toward the left in the figure), as illustrated in FIG. 8(a). In this case, the discharge of ink from the inkjet heads 50-2 to 50-5 is not carried out.

Thus, a layer 28-1 of the ink 2 formed in this manner is the same layer as the layer 24-1 of the ink 2 as illustrated in FIG. 5. Here, the biodegradable resin in the ink 2 is semi-dried (semi-cured) to the extent that it is not completely cross-linked.

Also in the ink mixing process, it is preferred that the above-mentioned forming process includes a process of semi-drying the layer discharged. For example, for a semi-dry process, it is preferred to maintain at an environmental temperature of 40°C to 120°C for a predetermined period of time after the discharge of ink, and it is more preferred to maintain at an environmental temperature of 50°C to 100°C for a predetermined period of time. The corresponding maintenance time is preferably is 10 seconds to 120 seconds, and more preferably 20 seconds to 90 seconds.

Next, the mixed ink supplied from the ink tank 60-2 by the inkjet head 50-2 (an ink in which the mixing ratio of the ink 1 and the ink 2 is 25:75) is discharged on the layer 28-1 of the ink 2 to form a mixed layer 28-2.

The formation of the mixed layer 28-2 is carried out by discharging the mixed ink by the inkjet head 50-2 while moving the stage 30 as illustrated in FIG. 8 (b). In the same manner as in the embodiment by the drawing mixing method, since the layer 28-1 of the ink 2 is in a semi-dried state, the solvent of the ink of the mixed layer 28-2 formed thereon is received in the layer 28-1 of the ink 2, and thus there is no extreme leakage and spreading. Therefore, the heating temperature needs to be adjusted with the ease of evaporation of the ink.

By semi-drying the mixed layer 28-2, the mixed layer 28-2 is in a state where the biodegradable resin and the cytokine contained in the ink 1 and the biodegradable resin and calcium phosphate contained in the ink 2 are stacked.

Moreover, a mixed layer 28-3 is formed on the mixed layer 28-2 by discharging the mixed ink (a mixed ink in which the mixing ratio of the ink 1 and the ink 2 is 50:50) supplied from ink tank 60-3 by the inkjet head 50-3 (not illustrated in FIG. 8).

Since the mixed layer 28-2 is in semi-dried state, the solvent of the ink of the mixed layer 28-3 formed thereon is received in the mixing layer 28-2. In addition, the mixed layer 28-3 is also semi-dried.

Thus, each mixed inks was discharged by laminating the mixed layers 28-2 to 28-4 in order of higher mixing ratio of the ink 2 (in order of lower mixing ratio of the ink 1), and the ink 1 (an ink in which the mixing ratio of the ink 1 and the ink 2 is 100:0) supplied from the ink tank 60-5 by the inkjet head 50-5 is finally discharged to form a layer 28-5 in which the ink 1 is 100% (a layer of the ink 1) (FIG. 8(c)).

After the completion of the formation of all the layers, a biodegradable resin film 2 having composition ratio from 100% of the ink 1 to100% of the ink 2 is formed as illustrated in FIG. 1. After the completion of the formation of all the layers, for a full-dry process, for example, it is preferred to maintain at an environmental temperature of 40°C to 120°C for a predetermined period of time after the discharge of ink, and it is more preferred to maintain at an environmental temperature of 50°C to 100°C for a predetermined period of time. The corresponding maintenance time is preferably 10 seconds to 120 seconds, and more preferably 20 seconds to 90 seconds. Here, the ratio of the cytokine and the calcium phosphate in each later is different. Accordingly, a biodegradable resin film 2 having the gradient structure in which the ratio of the calcium phosphate to the cytokine changes continuously from 100% to 0% from the side B to the side A. Further, since the concentration of the cross-linking agent in each layer is different, the degree of cross-linking of the biodegradable resin is different in each layer after a full-cure. As a result, in the biodegradable resin film 2, the gradient structure in which the degree of cross-linking of the biodegradable resin is changed continuously from 100% to 0% from the side B to the side A is obtained.

Further, in the step of forming each layer, from the viewpoint of stable discharge, the liquid amount of ink droplets discharged from the inkjet head is preferably from 0.5 pL to 150 pL, more preferably from 0.7 pL to 130 pL, and still more preferably from 1 pL to 100 pL.

In the step of forming each layer, from the viewpoint of good film-forming properties, the diameter of ink droplets discharged from the inkjet head is preferably from 2 µm to 450 µm, more preferably from 5 µm to 350 µm, and still more preferably from 10 µm to 250 µm.

As described above, a biodegradable resin film having a gradient structure was formed by using the mixed ink. According to the ink mixing method of the present embodiment, since it is well mixed at the step of the ink, it is possible to prepare a biodegradable resin film having a gradient structure in which the accuracy of the changes of the ratio of the cytokine and the calcium phosphate and the degree of cross-linking of the biodegradable resin is high. In addition, in comparison to the embodiment by the drawing mixing method, since the time to diffuse and mix two kinds of functional inks is unnecessary, there is an advantage that the process time can be shortened.

In the present embodiment, a mixed layer of the ink 1 and the ink 2 was formed in three layers, but the number of layers is not limited thereto. Any number of layers may be stacked as long as the layers are stacked such that the mixing ratio of each ink is inclined. Further, it is necessary to prepare ink tanks and inkjet heads as many as layers to be formed.

Further, in this embodiment, a biodegradable resin film having a gradient structure having composition ratio from 100% of the ink 2 to 100% of the ink 1 was formed, but it is not necessarily to adopt the composition ratio in which the ink 2 is 100% or the ink 1 is 100%, and as long as the gradient structure is obtained within the range, the composition ratio can be optionally changed.

The composition ratio may be adjusted appropriately depending on the characteristics of the biodegradation speed of the biodegradable resin film having a gradient structure to be obtained.

### Examples

Hereinafter, the examples of the present invention will be described more specifically, but the scope of the present invention may not be interpreted or somewhat limited thereto.

### <Example 1>

### (Preparation of tissue blocking layer forming ink (the second ink: Ink 2))

| - Blocking Ink A1- | |
|---|---|
| aqueous solution of 10% by mass of acidic gelatin (Manufactured by Nitta Gelatin Co., Ltd.) | 25g |
| calcium phosphate: β-TCP (Manufactured by Nanocube Japan Co., Ltd.) | 2.5g |
| glutaraldehyde (Manufactured by Nacalai Tesque Co., Ltd.; aqueous solution of 25% by mass) | 0.5g |
| Olfine E1010 (Manufactured by Nissin Chemical Industry Co., Ltd.) | 1g |
| ion-exchanged water | 200g |

The above materials were charged to the vessel of 500mL and the temperature of the liquid was kept below 40°C, and the mixture was stirred with a Silverson high-speed stirrer for 20 minutes. Then, the mixture was filtered through a filter of 10µm to prepare blocking ink A1.

### (Preparation of guided tissue regeneration layer forming ink (the first ink: Ink 1))

| - Regeneration Induction Ink B1- | |
|---|---|
| aqueous solution of 10% by weight of acidic gelatin (Manufactured by Nitta Gelatin Co., Ltd.) | 55g |
| cytokine: bFGF (Manufactured by Wako Pure Chemical Industries, Ltd.) | 0.001g |
| Olfine E1010 (Manufactured by Nissin Chemical Industry Co., Ltd.) | 1g |
| Ion-exchanged water | 200g |

The above-mentioned materials were charged to 500mL vessel and the temperature of the liquid was kept below 40°C, and the mixture was stirred with a Silverson high-speed stirrer for 5 minutes. Then, the mixture was filtered through a filter of 2 µm to prepare regeneration induction ink B 1.

A biodegradable resin film in a square of 15mm × 15mm and having a thickness of 100 µm was formed on the quartz glass substrate by an inkjet drawing method A to be described later by using the blocking ink A1 and the regeneration induction ink B1.

### <Inkjet Drawing Method A>

The regeneration induction ink B1 and the blocking ink A1 were respectively filled in ink tank 1, and the ink tank 2 as illustrated in FIG. 3. The inks supplied to the inkjet head 1 and the inkjet head 2 are the regeneration induction ink B1 and the blocking ink A1, respectively.

First, the droplet amount of ink droplets discharged from the inkjet head 2 was controlled to be 10 pL, and the droplet diameter was controlled to be 30 µm, and blocking ink A1 was discharged in a nitrogen gas atmosphere from the inkjet head 2. Here, an ink layer 1 was formed without discharging the regeneration induction ink B1 from the inkjet head 1 (i.e., the ratio (% by mass) of the discharge amount of ink discharged from the inkjet head 1 and the discharge amount of ink discharged from the inkjet head 2 of 100:0), dried at 80°C for 30 seconds, and then semi-cured.

Subsequently, a biodegradable resin film having a gradient structure in which the ratio of calcium phosphate to cytokine and the degree of cross-linking of the biodegradable resin (acidic gelatin) continuously decreases from the layer 1 side to the layer 5 side in a thickness direction was formed by changing the ratio (% by mass) of the discharge amount of ink discharged from the inkjet head 2 and the discharge amount of ink discharged from the inkjet head 1 to 75:25 (ink layer 2), 50:50 (ink layer 3), 25:75 (ink layer 4), 0:100 (ink layer 5), repeating lamination and semi-curing, and finally drying and total-curing at 80°C for 5 minutes.

Here, at the time of forming ink layer 2, the droplet amount of ink droplets of the regeneration induction ink B1 discharged from the inkjet head 1 was set to be 5 pL, the droplet diameter was set to be 20 µm, the droplet amount of ink droplets of the blocking ink A1 discharged from the inkjet head 2 was set to be 10 pL, and the droplet diameter was set to be 30 µm. At the time of forming ink layer 3, the droplet amount of ink droplets of the regeneration induction ink B1 was set to be 10 pL, the droplet diameter was set to be 30 µm, the droplet amount of ink droplets of the blocking ink A1 was set to be 10 pL, and the droplet diameter was set to be 30 µm. At the time of forming ink layer 4, the droplet amount of ink droplets of the regeneration induction ink B1 was set to be 10 pL, the droplet diameter was set to be 30 µm, the droplet amount of ink droplets of the blocking ink A1 was set to be 5 pL, and the droplet diameter was set to be 20 µm. At the time of forming ink layer 5, the droplet amount of ink droplets of the regeneration induction ink B1 was set to be 10 pL, and the droplet diameter was set to be 30 µm.

The film thicknesses of ink layers 1 to 5 after total curing were set to be 20 µm, respectively.

In addition, the ratio of calcium phosphate on cytokine in each layer was confirmed by the combined use of high-sensitivity mass spectrometer and XPS. Further, the degree of cross-linking of the biodegradable resin (acidic gelatin) in each layer was confirmed by determining the swelling degree capable of measuring the swollen film thickness of each layer with a swelling meter. As a result, the gradient structure was confirmed.

Thereafter, the formed biodegradable resin film was dried and hardened at room temperature for 12 hours, washed with 100 mM glycine solution while shaking for 1 hour, and then lyophilized.

Then, a biodegradable resin film was obtained as a tissue regeneration member by peeling off and isolating from the quartz glass substrate. The biodegradability and the film strength of the resulting biodegradable resin film were evaluated as described below. Further, the inkjet suitabilities of the used blocking ink A1 and regeneration induction ink B1 were also evaluated as described below.

### - Evaluation -

### <Biodegradability>

The formed biodegradable resin film was processed into a square of 5mm × 5mm, was radiolabeled with ¹²⁵I to the tyrosine residues of the gelatin molecules by the chloramine-T method, was buried subcutaneously under the dorsum of a mouse, and the gels and the surrounding tissue were excised over time, the residual radiation count was measured by calculating the residual ratio after 3 months and 6 months and evaluated according to the following criteria. (3 months later)
A: 40% or more of residual ratio
B: 20% or more and less than 40% of residual ratio
C: less than 20% of residual ratio (after 6 months)
A: less than 5% of residual ratio
B: 5% or more and less than 10% of residual ratio
C: 10% or more of residual ratio

### <Film strength>

The formed biodegradable resin film was processed into a rectangle of 10mm × 5mm, and was pulled up and down in the test speed of 20 mm/min by using a small desktop tensile tester EZ Test EZ-S type manufactured by Shimazu Seisakusho Co., Ltd. The strength test of the samples was carried out, and was evaluated according to the following criteria.
A: 10 mm or more of elongation
B: 5 mm or more and less than 10mm of elongation
C: less than 5 mm of elongation

<Inkjet Suitability>

The prepared blocking ink A1 and the regeneration induction ink B1 were respectively placed in "Material Printer DMP-2831" (manufactured by Fuji Photo Film Co., Ltd.), were discharged continuously in all 16 nozzles for 30 minutes by using 10 pl head, and were evaluated according to the following criteria about inkjet suitability of each ink (discharge reliability) by detecting the number of non-discharge nozzles after 30 minutes.
A: the number of non-discharge nozzles of 0 to 1
B: the number of non-discharge nozzles of 2 to 5
C: the number of non-discharge nozzles of 6 or more

The above-mentioned evaluation results of the biodegradable resin film, blocking ink A1, and the regeneration induction ink B1 formed in Example 1 are shown in Table 1.

### <Example 2>

A mixed ink G1 (the mixing ratio (% by mass) A1: B1 = 75:25), G2 (the mixing ratio (% by mass) A1: B1 = 50:50), G3 (the mixing ratio (% by mass) A1: B1 = 25:75) formed by mixing the blocking ink A1 and the regeneration induction ink B1 used in Example 1 were prepared and five kinds of inks including A1 and B1 were placed respectively on the quartz glass substrate in the order of A1 (bottom layer), G1, G2, G3, B1 (top layer) by using a total of five print heads to form a biodegradable resin film having a thickness of 100 µm in a square of 15 mm × 15 mm by an inkjet drawing method B to be described below.

### <Inkjet drawing method B>

The inks A1, G1, G2, G3, and B1 were respectively filled in the ink tanks 60-1 to 60-5 as illustrated in FIG. 7. The ink supplied to inkjet heads 50-1 to 50-5 are inks A1, G1, G2, G3, B1, respectively

First, the ink A1 was discharged from the inkjet head 50-1 in a nitrogen gas atmosphere, while controlling the droplet amount of ink droplets discharged from the inkjet head to be 10 pL and the droplet diameter of droplets to be 30µm.

The formed ink A1 layer was dried at 80°C for 30 seconds, and semi-cured.

Then, the ink G1 was discharged from the inkjet head 50-22 in the same manner, and the ink G1 layer was laminated and semi-cured. The inks G2, G3, B1 are also subjected to the lamination process and semi-cure process repeatedly, and finally dried and totally cured at 80°C for 5 minutes to form a biodegradable resin film having a gradient structure in which the ratio of calcium phosphate to cytokine and the degree of cross-linking of the biodegradable resin (acidic gelatin) decrease continuously from the layer A1 side toward the layer B1 side in the thickness direction.

In addition, the thicknesses of ink layers A1, G1, G2 G3, and B1 after full cure were set to be 20 µm, respectively.

The ratio of calcium phosphate on cytokine in each layer was confirmed by the combined use of high-sensitivity mass spectrometer and XPS. Further, the degree of cross-linking of the biodegradable resin (acidic gelatin) in each layer was confirmed by determining the swelling degree capable of measuring the swollen film thickness of each layer with a swelling meter. As a result, the gradient structure was confirmed.

Thereafter, the formed biodegradable resin film was dried and hardened at room temperature for 12 hours, washed with 100 mM glycine solution while shaking for 1 hour, and then lyophilized.

Then, a biodegradable resin film was obtained as a tissue regeneration member by peeling off and isolating from the quartz glass substrate. The biodegradability and the film strength of the resulting biodegradable resin film and the inkjet suitability of each ink were also evaluated as described below. The evaluated results were shown in Table 1.

### <Examples 3 to 11>

The blocking inks A2 to A8 and the regeneration induction inks B2 to B5 were prepared in the same manner as in Example 1 except that the biodegradable resin, the calcium phosphate and the cytokine contained in the blocking ink and the regeneration induction ink were replaced with those described in the following Tables 1 and 2.

The prepared blocking inks A2 to A8 and the regeneration induction inks B2 to B5 were used in a combination described in Tables 1 and 2 to form a biodegradable resin film in the same manner as in Example 1. The biodegradability and the film strength of the resulting biodegradable resin film and the inkjet suitability of each ink were evaluated in the same manner as in Example 1. The evaluated results were shown in Tables 1 and 2.

### <Comparative Example 1>

Only the regeneration induction ink B1 used in Example 1 was used to form a biodegradable resin film having a thickness of 100 µm in a square of 15 mm × 15 mm composed of only one layer on a quartz glass substrate by inkjet drawing method. Then, the obtained biodegradable resin film was treated in the same manner as in Example 1. The biodegradability and the film strength of the resulting biodegradable resin film and the inkjet suitability of the ink were evaluated in the same manner as in Example 1. The evaluated results were shown in Table 2.

### <Comparative Example 2>

The blocking ink A1 and the regeneration induction ink B1 used in Example 1 were used. The blocking ink A1 was inkjet drawn on a quartz glass substrate to form a film having a thickness of 100 µm in a square of 15 mm × 15 mm by the inkjet drawing, and after it is air-dried at room temperature for about 1 minute, the regeneration induction ink B1 was inkjet drawn to create a film (two-layer laminated film) having a thickness of 100 µm in a square of 15 mm × 15 mm. Then, the obtained biodegradable resin film was treated in the same manner as in Example 1. The biodegradability and the film strength of the resulting biodegradable resin film and the inkjet suitability of the ink were evaluated in the same manner as in Example 1. The evaluated results were shown in Table 2.

In Table 1 and Table 2, "VC" and "ST" described in the column of ink type refers to the viscosity (mPa·s) and the surface tension (mN/m) of each ink, respectively.

The measurement of the viscosity was performed by E-type viscometer (manufactured by Toki Sangyo Co., Ltd.).

The measurement of the surface tension was performed by Wilhelmy plate submerged surface tensiometer (manufactured by Kyowa Interface Science Co., Ltd. stock).

[Table 1]

**Table 1**

| Example No. | Ink types | | Ink material of tissue blocking layer | | | Ink material of guided tissue regeneration laver | | Biodegradability | | Film strength | Inkjet suitability | |
|---|---|---|---|---|---|---|---|---|---|---|---|---|
| | Blocking ink | Regeneration induction ink | Biodegradable resin Crosslinking agent | Calcium phosphate | Particle diameter (µm) | Biodegradable resin | Cytokine | 3 months | 6 months | | Blocking ink | Regeneration induction ink |
| 1 | A1 VC:10 ST:26 | B1 VC:8 ST:28 | Acidic gelatin (Nitta Gelatin Co., Ltd.) Cross-linking agent: glutaraldehyde (Nacalai tesque, Inc.) | β-TCP (Nano Cube Co., Ltd.) | 0.1 | Acidic gelatin (Nitta Gelatin Co., Ltd.) | bFGF (Wako Pure Chemical Industries Co., Ltd.) | A | A | A | A | A |
| 2 | A1 VC:10 ST:26 | B1 VC:8 ST:28 | Acidic gelatin (Nitta Gelatin Co., Ltd.) Cross-linking agent: glutaraldehyde (Nacalai tesque, Inc.) | β-TCP (Nano Cube Co., Ltd.) | 0.1 | Acidic gelatin (Nitta Gelatin Co., Ltd.) | bFGF (Wako Pure Chemical Industries Co., Ltd.) | A | A | A | A | A |
| 3 | A2 VC:12 ST:24 | B2 VC:10 ST:30 | Acidic gelatin (Nitta Gelatin Co., Ltd.) Cross-linking agent: glutaraldehyde (Nacalai tesque, Inc.) | β-TCP (Nano Cube Co., Ltd.) | 0.1 | Acidic gelatin (Nippi, Inc.) | bFGF (Wako Pure Chemical Industries Co., Ltd.) | A | A | A | A | A |
| 4 | A3 VC:8 ST:33 | B3 VC:6 ST:38 | Basic gelatin (Nitta Gelatin Co., Ltd.) Cross-linking agent: glutaraldehyde (Nacalai tesque, Inc.) | β-TCP (Nano Cube Co., Ltd.) | 0.1 | Basic gelatin (Nippi, Inc.) | bFGF (Wako Pure Chemical Industries Co., Ltd.) | A | A | A | A | A |

| Example No. | Ink types | | Ink material of tissue blocking layer | | | Ink material of guided tissue regeneration layer | | Biodegradability | | Film strength | Ink jet suitability | |
|---|---|---|---|---|---|---|---|---|---|---|---|---|
| | Blocking ink | Regeneration induction ink | Biodegradable resin Crosslinking agent | Calcium phosphate | Particle diameter (µm) | Biodegradable resin | Cytokine | 3 months | 6 months | | Blocking ink | Regeneration induction ink |
| 5 | A4 VC:6 ST:22 | B1 VC:8 ST:28 | RESOMER^{R} R 202H Poly(D,L-lactide) (SIGMA-ALDRICH) Cross-linking agent: glutaraldehyde (Nacalai tesque, Inc.) | β-TCP (Nano Cube Co., Ltd.) | 0.1 | Acidic gelatin (Nitta Gelatin Co. , Ltd.) | bFGF (Wako Pure Chemical Industries Co., Ltd.) | A | A | A | A | A |
| 6 | A5 VC:7 ST:20 | B1 VC:8 ST:28 | RESOMER^{R} R 502H Poly(D,L-lactide-co-glycolide) 50:50 (SIGMA-ALDRICH) Cross-linking agent: glutaraldehyde (Nacalai tesque, Inc.) | β-TCP (Nano Cube Co., Ltd.) | 0.1 | Acidic gelatin (Nitta Gelatin Co., Ltd.) | bFGF (Wako Pure Chemical Industries Co., Ltd.) | A | A | A | A | A |
| 7 | A6 VC:25 ST:35 | B1 VC:8 ST:28 | Alginic acid (Wako Pure Chemical Industries Co., Ltd.) Cross-linking agent: calcium carbonate (Wako Pure Chemical Industries Co., Ltd.) | β-TCP (Nano Cube Co., Ltd.) | 0.1 | Acidic gelatin (Nitta Gelatin Co., Ltd.) | bFGF (Wako Pure Chemical Industries Co., Ltd.) | A | A | A | A | A |
| 8 | A7 VC:14 ST:28 | B1 VC:8 ST:28 | Acidic gelatin (Nitta Gelatin Co., Ltd.) Cross-linking agent: glutaraldehyde (Nacalai tesque, Inc.) | Hydroxya patite (Nano Cube Co., Ltd.) | 0.08 | Acidic gelatin (Nitta Gelatin Co., Ltd.) | bFGF (Wako Pure Chemical Industries Co., Ltd.) | A | A | A | A | A |

[Table 2]

**Table 2**

| Example No. | Ink types | | Ink material of tissue blocking layer | | | Ink material of guided tissue regeneration layer | | Biodegradability | | Film strength | Ink jet suitability | |
|---|---|---|---|---|---|---|---|---|---|---|---|---|
| | Blocking ink | Regeneration induction ink | Biodegradable resin Crosslinking agent | Calcium phosphate | Particle diameter (µm) | Biodegradable resin | Cytokine | 3 months | 6 months | | Blocking ink | Regeneration induction ink |
| 9 | A1 VC:10 ST:26 | B4 VC:8 ST:28 | Acidic gelatin (Nitta Gelatin Co., Ltd.) Cross-linking agent: glutaraldehyde (Nacalai tesque, Inc.) | β-TCP (Nano Cube Co., Ltd.) | 0.1 | Acidic gelatin (Nitta Gelatin Co., Ltd.) | VEGF (Wako Pure Chemical Industries Co., Ltd.) | A | A | A | A | A |
| 10 | A1 VC:10 ST:26 | B5 VC:6 ST:24 | Acidic gelatin (Nitta Gelatin Co., Ltd.) Cross-linking agent: glutaraldehyde (Nacalai tesque, Inc.)) | β-TCP (Nano Cube Co., Ltd.) | 0.1 | RESOMER^{R} R 202H Poly(D,L-lactide) (SIGMA-ALDRICH) | bFGF (Wako Pure Chemical Industries Co., Ltd.) | A | A | A | A | A |
| 11 | A8 VC:11 ST:27 | B1 VC:8 ST:28 | Acidic gelatin (Nitta Gelatin Co., Ltd.) Cross-linking agent: glutaraldehyde (Nacalai tesque, Inc.) | β-TCP (Taihei Chemical Industrial Co., Ltd..) | 9 | Acidic gelatin (Nitta Gelatin Co., Ltd.) | bFGF (Wako Pure Chemical Industries Co., Ltd.) | A | A | A | A | A |

| Comp. Example No. | | | | | | | | | | | | |
|---|---|---|---|---|---|---|---|---|---|---|---|---|
| 1 | - | B1 VC:8 ST:28 | - | - | | Acidic gelatin (Nitta Gelatin Co., Ltd.) | bFGF (Wako Pure Chemical Industries Co., Ltd.) | C | A | A | - | A |
| 2 | A1 VC:10 ST:26 | B1 VC:8 ST:28 | Acidic gelatin (Nitta Gelatin Co., Ltd.) Cross-linking agent: glutaraldehyde (Nacalai tesque, Inc.) | β-TCP (Nano Cube Co., Ltd.) | 0.1 | Acidic gelatin (Nitta Gelatin Co., Ltd.) | bFGF (Wako Pure Chemical Industries Co., Ltd.) | B | C | C | A | A |

As shown in Table 1 and Table 2, Examples 1 to 13 have good biodegradability, strength and inkjet suitability, and a tissue regeneration member comprised of the biodegradable resin film having the gradient structure formed by various inkjet methods A (Drawing mixing method) and B (Ink mixing method) exhibits effective biodegradability and film strength in practical use. There is no difference in effects between two kinds of inkjet methods, and a tissue regeneration member having sufficient function may be formed by both methods. These results are due to form a gradient structure in which each composition is changed continuously. By using the tissue regeneration member of the present invention, the guided tissue regeneration method (GTR method) or the guided bone regeneration method (GBR method) was successful as a reproduction treatment of hard tissue (bone, teeth, etc.), which suggests that the space for the regeneration is ensured and the blocking of the entry of soft tissue can be achieved efficiently.

On the other hand, as in Comparative Example 1, in the case of forming a film by the normal inkjet drawing using only regeneration-inducing ink used for the present invention, the biodegradation rate is faster, and it is difficult to secure the time required for bone regeneration.

Further, since the blocking ink and the regeneration-inducing ink are laminated as they are in Comparative Example 2, the distortion is residual at the interface of two kinds of ink, the film strength is insufficient, and further the tissue blocking film is residual for a long period of time. Thus, it is concerned that the inflammation may occur by foreign objects even after the tissue regeneration.

### Industrial Applicability

The tissue regeneration member of the present invention and a method of forming the same can obtain the desired tissue regeneration capacity and the mechanical strength. Further, the ink of the present invention may be used to form a tissue regeneration member having the desired tissue regeneration capacity and the mechanical strength.

The present invention was described in detail with reference to specific embodiments, but it will be apparent to those of ordinary skill in the art that various changes and modifications can be carried out without departing from the spirit and scope of the present invention.

This application is based on Japanese Patent Application (Patent Application No. 2011-253214) filed on November 18, 2011, the contents of which are incorporated herein by reference.

### Description of Reference Numerals

- 1:: tissue regeneration member
- 2:: biodegradable resin film
- 10:: drawing unit
- 100:: biodegradable resin film production apparatus

## Claims

1. A tissue regeneration member comprising a biodegradable resin film containing cytokine and calcium phosphate, and having a gradient structure in which a ratio between the cytokine and the calcium phosphate increases or decreases continuously in a thickness direction.

2. The tissue regeneration member according to claim 1,
wherein in the gradient structure, the degree of cross-linking of the biodegradable resin is changed continuously in the thickness direction, the degree of cross-linking of the biodegradable resin is high at a side where the ratio of the calcium phosphate to the cytokine is high, and the degree of cross-linking of the biodegradable resin is low at a side where the ratio of the calcium phosphate to the cytokine is low.

3. The tissue regeneration member according to claim 1 or 2,
wherein the calcium phosphate is at least one kind of β-TCP, α-TCP, tetracalcium phosphate and hydroxyapatite.

4. The tissue regeneration member according to any one of claims 1 to 3,
wherein the cytokine is at least one kind of bFGF, VEGF, TGF-β, G-CSF, EPO, BMP-2, TGF-β1, cell growth factor in platelet and Angiopoietin.

5. The tissue regeneration member according to any one of claims 1 to 4,
wherein the calcium phosphate is a particle having an average particle size of 5 nm to 10 µm.

6. A method of forming a tissue regeneration member according to any one of claims 1 to 5, containing:
discharging a first ink containing a biodegradable resin and cytokine and a second ink containing a biodegradable resin and calcium phosphate on a substrate by an inkjet method to form, on the substrate, a biodegradable resin film having a gradient structure in which a ratio between the calcium phosphate and the cytokine increases or decreases continuously in a thickness direction.

7. The method of forming a tissue regeneration member according to claim 6,
wherein the inkjet method uses at least a first inkjet head and a second inkjet head, the method containing:
a step of supplying the first ink to the first inkjet head;
a step of supplying the second ink to the second inkjet head;
a controlling step of determining a ratio of an amount of the first ink discharged from the first inkjet head and an amount of the second ink discharged from the second inkjet head;
a forming step of discharging the first ink or the second ink from at least one of the first inkjet head and the second inkjet head depending on the determined ratio to form one layer; and
a laminating step of laminating a plurality of layers on the substrate through repeating the forming step to obtain a biodegradable resin film having the gradient structure,
wherein, in the controlling step, the ratio of the ink is determined such that the ratio of the first ink becomes higher and the ratio of the second ink becomes lower, from the closest layer towards the farthest layer from the substrate or from the farthest layer towards the closest layer to the substrate in the thickness direction of the plurality of layers.

8. The method of forming a tissue regeneration member according to claim 7,
wherein an ink amount of droplets discharged from the first and second inkjet heads is from 0.3 pL to 100 pL.

9. The method of forming a tissue regeneration member according to claim 7 or 8,
wherein a droplet diameter of droplets discharged from the first and second inkjet heads is from 1 µm to 300 µm.

10. The method of forming a tissue regeneration member of claim 6,
wherein the inkjet method uses a plurality of inkjet heads, the method containing:
a step of supplying a plurality of mixed inks including the first ink containing the first material and the second ink containing the second material which are mixed in different ratios, respectively, to each of the plurality of inkjet heads;
a selecting step of selecting inkjet heads one by one sequentially from the plurality of inkjet heads, the selecting being made sequentially from an inkjet head to which a mixed ink having a high ratio of the first or second ink is supplied to an inkjet head to which a mixed ink having a low corresponding ratio is supplied;
a forming step of discharging the mixed ink from the selected inkjet head to form a layer; and
a laminating step of laminating a plurality of layers on the substrate through repeating the forming step to obtain a biodegradable resin film having the gradient structure.

11. The method of forming a tissue regeneration member according to claim 10,
wherein an ink amount of droplets discharged from the first and second inkjet heads is from 0.5 pL to 150 pL.

12. The method of forming a tissue regeneration member according to claim 10 or 11,
wherein a droplet diameter of droplets discharged from the first and second inkjet heads is from 2 µm to 450 µm.

13. The method of forming a tissue regeneration member according to any one of claims 6 to 12,
wherein the first and second inks contain the biodegradable resin and the cytokine or the calcium phosphate, and the inks have a viscosity of 2 mPa·s to 50 mPa·s and a surface tension of 15 mN/m to 40 mN/m.

14. An ink containing a biodegradable resin, and cytokine or calcium phosphate,
wherein the ink has a viscosity of 2 mPa·s to 50 mPa·s and a surface tension of 15 mN/m to 40 mN/m.
